# EUROPEAN PATENT SPECIFICATION

(11) **EP 2 861 788 B1**
(45) Date of publication and mention of the grant of the patent: **10.10.2018**
(21) Application number: 13803970.6
(22) Date of filing: 14.06.2013
(51) Int. Cl.: C40B 40/04, G01N 33/574, G01N 33/569, G01N 33/68, C12Q 1/68

(54) **METHODS OF DETECTING DISEASES OR CONDITIONS USING CIRCULATING DISEASED CELLS**
VERFAHREN ZUM NACHWEIS VON KRANKHEITEN ODER LEIDEN MITTELS ZIRKULIERENDER ERKRANKTER ZELLEN
MÉTHODES DE DÉTECTION DE MALADIES OU D'ÉTATS AU MOYEN DE CELLULES INFECTÉES EN CIRCULATION

(30) Priority: 15.06.2012 US 201261660518 P
(43) Date of publication of application: 22.04.2015
(73) Proprietor: Progenity, Inc., San Diego, CA 92122 (US)
(72) Inventor: STYLLI, Harry, La Jolla, CA 92037 (US)
(74) Representative: Vossius & Partner Patentanwälte Rechtsanwälte mbB
(86) International application number: PCT/US2013/045991
(87) International publication number: WO 2013/188828

(56) References cited:
- EP-A1- 1 542 016
- US-A1- 2003 113 910
- US-A1- 2005 181 463
- US-A1- 2011 110 931
- US-A1- 2012 053 073
- US-A1- 2012 053 073
- D. A. SMIRNOV: "Global Gene Expression Profiling of Circulating Tumor Cells", CANCER RESEARCH, vol. 65, no. 12, 15 June 2005 (2005-06-15) , pages 4993-4997, XP055081859, ISSN: 0008-5472, DOI: 10.1158/0008-5472.CAN-04-4330
- ELIZABETH A. PUNNOOSE ET AL: "Molecular Biomarker Analyses Using Circulating Tumor Cells", PLOS ONE, vol. 5, no. 9, 1 January 2010 (2010-01-01) , page e12517, XP055031075, ISSN: 1932-6203, DOI: 10.1371/journal.pone.0012517
- PUTAO CEN ET AL: "Circulating tumor cells in the diagnosis and management of pancreatic cancer", BBA - REVIEWS ON CANCER, ELSEVIER SCIENCE BV, AMSTERDAM, NL, vol. 1826, no. 2, 31 May 2012 (2012-05-31) , pages 350-356, XP028516760, ISSN: 0304-419X, DOI: 10.1016/J.BBCAN.2012.05.007 [retrieved on 2012-06-07]
- ROSA NADAL ET AL: "Biomarkers characterization of circulating tumour cells in breast cancer patients", BREAST CANCER RESEARCH, CURRENT SCIENCE, LONDON, GB, vol. 14, no. 3, 3 May 2012 (2012-05-03), page R71, XP021096236, ISSN: 1465-5411, DOI: 10.1186/BCR3180
- ANDREIA DE ALBUQUERQUE ET AL: "Multimarker Analysis of Circulating Tumor Cells in Peripheral Blood of Metastatic Breast Cancer Patients: A Step Forward in Personalized Medicine", BREAST CARE, vol. 7, no. 1, 1 January 2012 (2012-01-01) , pages 7-12, XP055250030, CH ISSN: 1661-3791, DOI: 10.1159/000336548
- DIJKSTRA G ET AL: "Increased expression of inducible nitric oxide synthase in circulating monocytes from patients with active inflammatory bowel disease", SCANDINAVIAN JOURNAL OF GASTROENTEROLOGY, vol. 37, no. 5, May 2002 (2002-05), pages 546-554, XP008179004, ISSN: 0036-5521
- BURAKOFF ROBERT ET AL: "Whole blood gene expression differentiates between active disease and remission in patients with Crohn's disease", GASTROENTEROLOGY, vol. 130, no. 4, Suppl. 2, April 2006 (2006-04), page A201, XP008179008, & DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 -24, 2006 ISSN: 0016-5085

## Description

### Field of the Invention

This invention relates generally to methods of using circulating diseased cells in the diagnosis, prognosis, or monitoring of a disease or condition. The disclosure also relates to methods of using circulating diseased cells to identify markers of diseases or conditions.

### Background of the Invention

Early diagnosis of a disease often increases the likelihood of successful treatment or cure of such disease. Current diagnostic methods, however, depend largely on population-derived average values obtained from healthy individuals. Personalized diagnostic methods are needed that enable the diagnosis, especially the early diagnosis, of the presence of a disease or a condition in individuals who are not known to have the disease or who have recurrent disease. An in vitro method for the screening of anti-HIV compounds, or for predicting the progression of HIV related diseases, or for determining the efficiency of an anti-HIV-1 treatment based on the measuring of the expression level of Puma, and, optionally, on the measuring of the phosphorylated p53 and/or the p38 kinase protein level in circulating white blood cells of a sample from a HIV infected patient is disclosed in EP 1 542 016. Furthermore, Dijkstra et al. (2002) Scandinavian Journal of Gastroenterology 37, no. 5, pp. 546-554 discloses increased expression of inducible nitric oxide synthase in circulating monocytes from patients with active inflammatory bowel disease. In addition, Burakoff et al. (2006) Gastroenterology 130, no. 4, page A201, discloses that whole blood gene expression differentiates between active disease and remission in patients with Crohn's disease.

One object of the present invention is to provide diagnostic methods that can facilitate the detection of a disease or condition-specific markers, e.g., nucleic acids, proteins, carbohydrates, and/or lipids and the like by using circulating diseased cells. Another object of this invention is to provide methods of identifying a disease or condition-specific markers and further use such markers alone or together with any known markers to diagnose diseases or conditions.

### Summary of the Invention

Some embodiments of the invention are:
1. A method for diagnosing or aiding in the diagnosis of a disease or condition in a subject comprising:
   a) determining a first profile of one or more markers of the disease or condition from a population of circulating diseased cells isolated from the subject, wherein the circulating diseased cells are affected by the disease or condition, and wherein the circulating diseased cells are blood cells;
   b) determining a second profile of at least one of the one or more markers from a population of control cells or a control bodily fluid sample isolated from the subject, wherein the control cells or the control bodily fluid sample are at least 75% free of cells affected by the disease or condition; and
   c) identifying a difference between the first and second profiles, wherein the difference is indicative of the presence of said disease or condition, or the risk of developing said disease or condition, or the prognosis of said disease or condition, in the subject.
2. A method for assessing the risk of developing a disease or condition in a subject is also disclosed, the method comprising:
   a) determining a first profile of one or more markers of the disease or condition from a population of circulating diseased cells isolated from the subject, wherein the circulating diseased cells are affected by the disease or condition;
   b) determining a second profile of at least one of the one or more markers from a population of control cells or a control bodily fluid sample isolated from the subject, wherein the control cells or the control bodily fluid sample are substantially free of cells affected by the disease or condition; and
   c) identifying a difference between the first and second profiles, wherein the difference is indicative of the risk of developing said disease or condition in the subject.
3. A method for prognosing or aiding in the prognosis of a disease or condition in a subject is also disclosed, the method comprising:
   a) determining a first profile of one or more markers of the disease or condition from a population of circulating diseased cells isolated from the subject, wherein the circulating diseased cells are affected by the disease or condition;
   b) determining a second profile of at least one of the one or more markers from a population of control cells or a control bodily fluid sample isolated from the subject, wherein the control cells or the control bodily fluid sample are substantially free of cells affected by the disease or condition; and
   c) identifying a difference between the first and second profiles, wherein the difference is indicative of the prognosis of said disease or condition in the subject.
4. A method for assessing the efficacy of a treatment for a disease or condition in a subject is also disclosed, the method comprising:
   a) determining a first profile of one or more markers of the disease or condition from a population of circulating diseased cells isolated from the subject before the treatment, wherein the circulating diseased cells are affected by the disease or condition;
      determining a second profile of at least one of the one or more markers from a population of control cells or a control bodily fluid sample isolated from the subject before the treatment, wherein the control cells or the control bodily fluid sample are substantially free of cells affected by the disease or condition;
      identifying a difference between the first and second profiles;
   b) determining a third profile of the one or more markers from a population of circulating diseased cells isolated from the subject after the treatment, wherein the circulating diseased cells are affected by the disease or condition;
      determining a fourth profile of at least one of the one or more markers from a population of control cells or a control bodily fluid sample isolated from the subject after the treatment, wherein the control cells or the control bodily fluid sample are substantially free of cells affected by the disease or condition;
      identifying a difference between the third and fourth profiles; and
   c) identifying a difference between the difference identified in a) and the difference identified in b), wherein the difference identified in c) is indicative of the efficacy of the treatment for said disease or condition in the subject.
5. A method for monitoring the progression or regression of a disease or condition in a subject is also disclosed, the method comprising:
   a) determining a first profile of one or more markers of the disease or condition from a population of circulating diseased cells isolated from the subject at a first time point, wherein the circulating diseased cells are affected by the disease or condition;
      determining a second profile of at least one of the one or more markers from a population of control cells or a control bodily fluid sample isolated from the subject at the first time point, wherein the control cells or the control bodily fluid sample are substantially free of cells affected by the disease or condition;
      identifying a difference between the first and second profiles;
   b) determining a third profile of the one or more markers from a population of circulating diseased cells isolated from the subject at a second time point, wherein the circulating diseased cells are affected by the disease or condition;
      determining a fourth profile of at least one of the one or more markers from a population of control cells or a control bodily fluid sample isolated from the subject at the second time point, wherein the control cells or the control bodily fluid sample are substantially free of cells affected by the disease or condition;
      identifying a difference between the third and fourth profiles; and
   c) identifying a difference between the difference identified in a) and the difference identified in b), wherein the difference identified in c) is indicative of the progression or regression of said disease or condition in the subject.
6. A method for identifying a compound capable of ameliorating or treating a disease or condition in a subject is also disclosed, the method comprising:
   a) determining a first profile of one or more markers of the disease or condition from a population of circulating diseased cells isolated from the subject before administering the compound to the subject, wherein the circulating diseased cells are affected by the disease or condition;
      determining a second profile of at least one of the one or more markers from a population of control cells or a control bodily fluid sample isolated from the subject before administering the compound to the subject, wherein the control cells or the control bodily fluid sample are substantially free of cells affected by the disease or condition;
      identifying a difference between the first and second profiles;
   b) determining a third profile of the one or more markers from a population of circulating diseased cells isolated from the subject after the administration of the compound, wherein the circulating diseased cells are affected by the disease or condition;
      determining a fourth profile of at least one of the one or more markers from a population of control cells or a control bodily fluid sample isolated from the subject after the administration of the compound, , wherein the control cells or the control bodily fluid sample are substantially free of cells affected by the disease or condition;
      identifying a difference between the third and fourth profiles;
   c) identifying a difference between the difference identified in a) and the difference identified in b), wherein the difference identified in c) indicates that the compound is capable of ameliorating or treating said disease or condition in the subject.
7. The method of any one of embodiments 1-6, wherein the circulating diseased cells are enucleated is also disclosed.
8. The method of any one of embodiments 1-7, wherein the control cells are enucleated is also disclosed.
9. The method of any one of embodiment 7 or 8, wherein the circulating diseased cells or control cells are enucleated using physical removal, chemical treatments, photo ablation, or ultraviolet irradiation is also disclosed.
10. The method of embodiment 9, wherein the physical removal uses a microneedle, optical tweezers, or aspiration is also disclosed.
11. The method of any one of embodiments 1-6, wherein at least one of the one or more markers is up-regulated or activated in the circulating diseased cells compared to the control cells is also disclosed.
12. The method of any one of embodiments 1-6, wherein at least one of the one or more markers is down-regulated or inhibited in the circulating diseased cells compared to the control cells is also disclosed.
13. The method of any one of embodiments 1-6, further comprising lysing the circulating diseased cells or the control cells before a) is also disclosed.
14. The method of any one of embodiments 1-6 and 13, further comprising extracting at least some of cellular contents from the circulating diseased cells or the control cells before a) is also disclosed.
15. The method of embodiment 14, wherein at least one of the one or more markers of said disease or condition is present in the cellular contents of the circulating diseased cells is also disclosed.
16. The method of embodiment 14, wherein the one or more markers of said disease or condition is not present in the cellular contents of the control cells is also disclosed.
17. The method of any one of embodiments 1-6, further comprising comparing the difference identified in c) to a repository of one or more known markers of said disease or condition is also disclosed.
18. The method of embodiment 17, wherein the repository is obtained by data mining is also disclosed.
19. The method of any one of embodiments 1-6, wherein the circulating diseased cells are isolated from a bodily fluid sample from the subject is also disclosed.
20. The method of embodiment 19, wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, buccal swab sample, sputum, bronchial lavage, Pap smear sample, or ocular fluid is also disclosed.
21. The method of any one of embodiments 1-6, wherein the circulating diseased cells are blood cells, tumor cells, lymphoma cells, fetal cells, apoptotic cells, epithelia cells, endothelial cells, stem cells, progenitor cells, mesenchymal cells, osteoblast cells, osteocytes, hematopoietic stem cells, foam cells, adipose cells, transcervical cells, circulating cardiocytes, circulating fibrocytes, circulating cancer stem cells, circulating myocytes, circulating cells from kidney, circulating cells from gastrointestinal tract, circulating cells from lung, circulating cells from reproductive organs, circulating cells from central nervous system, circulating hepatic cells, circulating cells from spleen, circulating cells from thymus, circulating cells from thyroid , circulating cells from an endocrine gland, circulating cells from parathyroid, circulating cells from pituitary, circulating cells from adrenal gland, circulating cells from islets of Langerhans, circulating cells from pancreas, circulating cells from hypothalamus, circulating cells from prostate tissues, circulating cells from breast tissues, circulating cells from circulating retinal cells, circulating ophthalmic cells, circulating auditory cells, circulating epidermal cells, circulating cells from the urinary tract, or mixtures thereof is also disclosed.
22. The method of any one of embodiments 1-6, wherein the circulating diseased cells are infected by an infectious agent is also disclosed.
23. The method of embodiment 22, wherein the infectious agent is a virus, bacteria, fungus, parasite, protozoan, infectious protein or microorganism is also disclosed.
24. The method of any one of embodiments 1-6, wherein the control cells are normal cells is also disclosed.
25. The method of any one of embodiments 1-6, wherein the control cells are circulating cells is also disclosed.
26. The method of any one of embodiments 1-6, wherein the circulating diseased cells are isolated using antibodies is also disclosed.
27. The method of any one of embodiments 1-6, wherein the circulating diseased cells are isolated by flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platform, or a combination thereof is also disclosed.
28. The method of any one of embodiments 1-6, wherein the circulating diseased cells are isolated by using a product secreted by the circulating diseased cells is also disclosed.
29. The method of any one of embodiments 1-6, wherein the circulating diseased cells are isolated by using a cell surface target on the surface of the circulating diseased cells is also disclosed.
30. The method of embodiment 29, wherein the target is expressed by the circulating diseased cells is also disclosed.
31. The method of embodiment 29, wherein the target is a marker of said disease or condition is also disclosed.
32. The method of any one of embodiments 1-6, wherein the circulating diseased cells are isolated using a ligand that binds to a molecular receptor expressed on the plasma membranes of the circulating diseased cells is also disclosed.
33. The method of embodiment 1, wherein the one or more markers are nucleic acids, proteins, lipids, carbohydrates, metabolites, or combinations thereof.
34. The method of embodiment 33, wherein the nucleic acids are nucleotides, oligonucleotides, DNAs, RNAs, or DNA-RNA hybrids is also disclosed.
35. The method of embodiment 34, wherein the DNAs are double-stranded DNAs, single-stranded DNAs, multi-stranded DNAs, complementary DNAs, genomic DNAs, or non-coding DNAs is also disclosed.
36. The method of embodiment 34, wherein the RNAs are messenger RNAs (mRNAs), microRNAs (miRNAs), small nucleolar RNAs (snoRNAs), ribosomal RNAs (rRNAs), transfer RNAs (tRNAs), small interfering RNAs (siRNAs), heterogeneous nuclear RNAs (hnRNAs), or small hairpin RNAs (shRNAs) is also disclosed.
37. The method of embodiment 33, wherein the proteins are amino acids, peptides, enzymes, antigens, antibodies, cytokines, lipoproteins, glycoproteins, or hormones is also disclosed.
38. The method of embodiment 33, wherein the lipids are fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lyso-sphingomyelin,, gangliosides, plasmalogen, sulfatide, low density lipoproteins (LDLs), very low density lipoproteins (VLDLs), high density lipoproteins (HDLs), sphingoid base-1-phosphates, or derivatives thereof is also disclosed.
39. The method of embodiment 33, wherein the carbohydrates are monosaccharides, disaccharides, polysaccharides, oligosaccharides, or derivatives thereof is also disclosed.
40. The method of embodiment 33, wherein the metabolites are primary metabolites, secondary metabolites, organic metabolites, inorganic metabolites, prostaglandins, hydroxyeicosatetraenoic acids, hydroxyoctadecadienoic acids, steroids, bile acids, vitamins, or derivatives thereof is also disclosed.
41. The method of embodiment 1, wherein the profile is a nucleic acid profile, a protein profile, a lipid profile, a carbohydrate profile, a metabolite profile, or a combination thereof.
42. The method of embodiment 41, wherein the profile is determined by a qualitative assay, a quantitative assay, or a combination thereof is also disclosed.
43. The method of embodiment 42, wherein the quantitative assay uses sequencing, direct sequencing, random shotgun sequencing, Sanger dideoxy termination sequencing, targeted sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof is also disclosed.
44. The method of embodiment 41, wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof.
45. The method of embodiment 44, wherein the nucleic acid profile is determined by polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), co-amplification at lower denaturation temperature-PCR (COLD-PCR), multiplex PCR, allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, bisulfite-driven conversion of non-methylated cytosine to uracil, methyl-binding PCR analysis, or a combination thereof is also disclosed.
46. The method of embodiment 44, wherein the nucleic acid profile is determined by a sequencing technique selected from the group consisting of direct sequencing, random shotgun sequencing, Sanger dideoxy termination sequencing, targeted sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof is also disclosed.
47. The method of embodiment 41, wherein the protein profile is a protein expression profile, a protein activation profile, or a combination thereof.
48. The method of embodiment 47, wherein the protein profile is determined by an immunohistochemistry assay, an enzyme-linked immunosorbent assay (ELISA), in situ hybridization, chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microscopy, microfluidic chip-based assays, surface plasmon resonance, sequencing, Western blotting assay, or a combination thereof is also disclosed.
49. The method of embodiment 47, wherein the protein activation profile comprises determining a phosphorylation state, an ubiquitination state, a myristoylation state, a conformational state, or a combination thereof of the one or more markers.
50. The method of embodiment 41, wherein the lipid profile is determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof is also disclosed.
51. The method of embodiment 41, wherein the carbohydrate profile is determined by chromatography, liquid chromatography, size exclusion chromatography, high performance anion exchange chromatography with pulsed amperometric detection (HPAEC-PAD), liquid chromatography, gas chromatography, fluorescent assay, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassay, microfluidic chip-based assay, detection of fluorescence, detection of chemiluminescence, or a combination thereof is also disclosed.
52. The method of any one the embodiments 1-6, wherein the subject has at least two diseases or conditions is also disclosed.
53. The method of any one the embodiments 1-6, wherein the subject is a mammal is also disclosed.
54. The method of embodiment 53, wherein the subject is a human is also disclosed.
55. The method of any one the embodiments 1-6, wherein the difference is greater than a 1-fold difference is also disclosed.
56. The method of embodiment 55, wherein the difference is at least 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference is also disclosed.
57. The method of any one of embodiments 1, 33, 41, 44, 47 or 49, wherein the disease or condition is a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a pediatric disease, disorder or condition, a mitochondrial disorder, a respiratory-gastrointestinal tract disease or condition, a reproductive disease or condition, an ophthalmic disease or condition, a musculo-skeletal disease or condition, or a dermal disease or condition.
58. A method for identifying one or more markers of a disease or condition is also disclosed, the method comprising:
   determining a first profile of analytes from a population of circulating diseased cells from a subject having said disease or condition, wherein the circulating diseased cells are affected by said disease or condition;
   determining a second profile of analytes from a population of control cells or a control bodily fluid sample isolated from the subject, wherein the control cells or the control bodily fluid sample are substantially free of cells affected by the disease or condition;
   identifying one or more analytes specific to the first profile relative to the second profile, the identified analytes being markers of said disease or condition.
59. The method of embodiment 58, wherein the circulating diseased cells are enucleated is also disclosed.
60. The method of embodiment 58, wherein the control cells are enucleated is also disclosed.
61. The method of embodiment 59 or 60, wherein the circulating diseased cells or control cells are enucleated using physical removal, chemical treatments, photo ablation, or ultraviolet irradiation is also disclosed.
62. The method of embodiment 61, wherein the physical removal uses a microneedle, optical tweezers, or aspiration is also disclosed.
63. The method of embodiment 58, further comprising lysing the circulating diseased cells or the control cells before a) is also disclosed.
64. The method of embodiment 58, further comprising extracting at least some of the cellular contents from the circulating diseased cells or the control cells before a) is also disclosed.
65. The method of embodiment 58, wherein the circulating diseased cells are isolated from a bodily fluid sample from the subject is also disclosed.
66. The method of embodiment 65, wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, buccal swab sample, sputum, bronchial lavage, Pap smear sample, or ocular fluid is also disclosed.
67. The method of embodiment 58, wherein the circulating diseased cells are blood cells, tumor cells, lymphoma cells, fetal cells, apoptotic cells, epithelia cells, endothelial cells, stem cells, progenitor cells, mesenchymal cells, osteoblast cells, osteocytes, hematopoietic stem cells, foam cells, adipose cells, transcervical cells, circulating cardiocytes, circulating fibrocytes, circulating cancer stem cells, circulating myocytes, circulating cells from kidney, circulating cells from gastrointestinal tract, circulating cells from lung, circulating cells from reproductive organs, circulating cells from central nervous system, circulating hepatic cells, circulating cells from spleen, circulating cells from thymus, circulating cells from thyroid, circulating cells from an endocrine gland, circulating cells from parathyroid, circulating cells from pituitary, circulating cells from adrenal gland, circulating cells from islets of Langerhans, circulating cells from pancreas, circulating cells from hypothalamus, circulating cells from prostate tissues, circulating cells from breast tissues, circulating cells from circulating retinal cells, circulating ophthalmic cells, circulating auditory cells, circulating epidermal cells, circulating cells from the urinary tract, or mixtures thereof is also disclosed.
68. The method of embodiment 58, wherein the circulating diseased cells are infected by an infectious agent is also disclosed.
69. The method of embodiment 68, wherein the infectious agent is a virus, bacteria, fungus, parasite, protozoan, infectious protein or microorganism is also disclosed.
70. The method of embodiment 58, wherein the control cells are normal cells is also disclosed.
71. The method of embodiment 58, wherein the control cells are circulating cells is also disclosed.
72. The method of embodiment 58, wherein the circulating diseased cells are isolated by using antibodies is also disclosed.
73. The method of embodiment 58, wherein the circulating diseased cells are isolated by flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platform, or a combination thereof is also disclosed.
74. The method of embodiment 58, wherein the circulating diseased cells are isolated using antibodies is also disclosed.
75. The method of embodiment 58, wherein the circulating diseased cells are isolated by using a product secreted by the circulating diseased cells is also disclosed.
76. The method of embodiment 58, wherein the circulating diseased cells are isolated by using a cell surface target on the surface of the circulating diseased cells is also disclosed.
77. The method of embodiment 76, wherein the target is expressed by the circulating diseased cells is also disclosed.
78. The method of embodiment 76, wherein the target is not expressed by the circulating diseased cells is also disclosed.
79. The method of embodiment 76, wherein the target is a marker of said disease or condition is also disclosed.
80. The method of embodiment 58, wherein the one or more markers are nucleic acids, proteins, lipids, carbohydrates, metabolites, or combinations thereof is also disclosed.
81. The method of embodiment 58, wherein the analytes are nucleic acids, proteins, lipids, carbohydrates, metabolites, or combinations thereof is also disclosed.
82. The method of embodiment 80 or 81, wherein the nucleic acids are nucleotides, oligonucleotides, DNAs, RNAs, or DNA-RNA hybrids.
83. The method of embodiment 82, wherein the DNAs are double-stranded DNAs, single-stranded DNAs, multi-stranded DNAs, complementary DNAs, genomic DNAs or non-coding DNAs is also disclosed.
84. The method of embodiment 82, wherein the RNAs are messenger RNAs (mRNAs), microRNAs (miRNAs), small nucleolar RNAs (snoRNAs), ribosomal RNAs (rRNAs), transfer RNAs (tRNAs), small interfering RNAs (siRNAs), heterogeneous nuclear RNAs (hnRNAs), or small hairpin RNAs (shRNAs) is also disclosed.
85. The method of embodiment 80 or 81, wherein the proteins are amino acids, peptides, enzymes, antigens, antibodies, cytokines, lipoproteins, glycoproteins, or hormones is also disclosed.
86. The method of embodiment 80 or 81, wherein the lipids are fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lyso-sphingomyelin, gangliosides, plasmalogen, sulfatide, low density lipoproteins (LDLs), very low density lipoproteins (VLDLs), high density lipoproteins (HDLs), sphingoid base-1-phosphates or derivatives thereof is also disclosed.
87. The method of embodiment 80 or 81, wherein the carbohydrates are monosaccharides, disaccharides, polysaccharides, oligosaccharides, or derivatives tehreof is also disclosed.
88. The method of embodiment 80 or 81, wherein the metabolites are primary metabolites, secondary metabolites, organic metabolites, inorganic metabolites, prostaglandins, hydroxyeicosatetraenoic acids, hydroxyoctadecadienoic acids, steroids, bile acids, vitamins, or derivatives thereof is also disclosed.
89. The method of embodiment 58, wherein the profile is a nucleic acid profile, a protein profile, a lipid profile, a carbohydrate profile, a metabolite profile, or a combination thereof is also disclosed.
90. The method of embodiment 89, wherein the profile is determined by a qualitative assay, a quantitative assay, or a combination thereof is also disclosed.
91. The method of embodiment 90, wherein the quantitative assay uses sequencing, targeted sequencing, single molecule real-time sequencing, electron microscopy-based sequencing, transistor-mediated sequencing, direct sequencing, random shotgun sequencing, Sanger dideoxy termination sequencing, targeted sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof is also disclosed.
92. The method of embodiment 89, wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof is also disclosed.
93. The method of embodiment 92, wherein the nucleic acid profile is determined by polymerase chain reaction (PCR) analysis, sequencing analysis, electrophoretic analysis, restriction fragment length polymorphism (RFLP) analysis, Northern blot analysis, reverse-transcriptase-PCR analysis (RT-PCR), co-amplification at lower denaturation temperature-PCR (COLD-PCR), multiplex PCR, quantitative PCR, quantitative RT-PCR, allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, surface plasmon resonance, bisulfite-driven conversion of non-methylated cytosine to uracil, methyl-binding PCR analysis, or a combination thereof is also disclosed.
94. The method of embodiment 92, wherein the nucleic acid profile is determined by a sequencing technique selected from targeted sequencing, single molecule real-time sequencing, electron microscopy-based sequencing, transistor-mediated sequencing, direct sequencing, random shotgun sequencing, Sanger dideoxy termination sequencing, targeted sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof is also disclosed.
95. The method of embodiment 89, wherein the protein profile is a protein expression profile, a protein activation profile, or a combination thereof is also disclosed.
96. The method of embodiment 95, wherein the protein activation profile comprises determining a phosphorylation state, an ubiquitination state, a myristoylation state, or a conformational state of the one or more markers is also disclosed.
97. The method of embodiment 95, wherein the protein profile is determined by an immunohistochemistry assay, an enzyme-linked immunosorbent assay (ELISA), chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, surface plasmon resonance, microfluidic chip-based assays, Western blotting assay, or a combination thereof is also disclosed.
98. The method of embodiment 89, wherein the lipid profile is determined by chromatography, liquid chromatography, size exclusion chromatography, high performance liquid chromatography (HPLC), gas chromatography, mass spectrometry, matrix assisted laser desorption/ionization-time of flight (MALDI-TOF) mass spectrometry, tandem mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assays, detection of fluorescence, detection of chemiluminescence, or a combination thereof is also disclosed.
99. The method of embodiment 89, wherein the carbohydrate profile is determined by chromatography, liquid chromatography, size exclusion chromatography, high performance anion exchange chromatography with pulsed amperometric detection (HPAEC-PAD), liquid chromatography, gas chromatography, fluorescent assay, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assays, detection of fluorescence, detection of chemiluminescence, or a combination thereof is also disclosed.
100. The method of embodiment 58, wherein the subject is a mammal is also disclosed.
101. The method of embodiment 100, where in the subject is a human is also disclosed.
102. The method of embodiment 58, wherein the disease or condition is a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a pediatric disease, disorder, or condition, a mitochondrial disorder, a respiratory-gastrointestinal tract disease or condition, a reproductive disease or condition, an ophthalmic disease or condition, a musculo-skeletal disease or condition, or a dermal disease or condition is also disclosed.
103. The method of embodiment 58, wherein the difference is greater than a 1-fold difference is also disclosed.
104. The method of embodiment 102, wherein the difference is at least 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference is also disclosed.
105. The method of embodiment 1, further comprising determining at least one diagnostic parameter of said disease or condition is also disclosed.
106. The method of embodiment 105, wherein the diagnostic parameter is determined by physical inspection, visual inspection, biopsy, scanning, histology, radiology, imaging, ultrasound, use of a commercial kit, genetic testing, immunological testing, analysis of bodily fluids, or monitoring neural activity is also disclosed.
107. The method of any one of embodiments 1-6, wherein the one or more markers comprise at least one or more of the markers identified by the method of embodiment 58 is also disclosed.
108. A kit comprising a plurality of marker detection agents that detect at least one or more of the markers identified by the method of embodiment 58 is also disclosed.
109. A method for treating or preventing a disease or condition in a subject comprising administering to said subject a composition comprising a compound identified by the method of embodiment 6 is also disclosed.
110. The method of any one of embodiments 1-6 and 58, wherein the control bodily fluid sample is a cell-free bodily fluid isolated from a bodily fluid is also disclosed.
111. The method of embodiment 110, wherein the cell-free bodily fluid is plasma or serum is also disclosed.
112. The method of embodiment 110, wherein the bodily fluid is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, buccal swab sample, sputum, bronchial lavage, Pap smear sample, or ocular fluid is also disclosed.

### Detailed Description of the Invention

Unless otherwise defined herein, scientific and technical terms used in this application shall have the meanings that are commonly understood by those of ordinary skill in the art. Generally, nomenclature used in connection with, and techniques of, cell and tissue culture, molecular biology, cell and cancer biology, neurobiology, neurochemistry, virology, immunology, microbiology, pharmacology, genetics and protein and nucleic acid chemistry, described herein, are those well known and commonly used in the art.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to". "Including" and "including but not limited to" are used interchangeably.

A "patient", "subject", or "individual" are used interchangeably and refer to either a human or a non-human animal. These terms include mammals, such as humans, primates, livestock animals (e.g., bovines, porcines), companion animals (e.g., canines, felines) and rodents (e.g., mice and rats).

As used herein, a control subject refers to any individual that has not been diagnosed as having the disease or condition being assayed. The terms "normal control", "healthy control", and "not-diseased cells" likewise mean a sample (e.g., cells, serum, tissue) taken from a source (e.g., subject, control subject, cell line) that does not have the condition or disease being assayed and therefore may be used to determine the baseline for the condition or disorder being measured. It is also understood that the control subject, normal control, and healthy control, include data obtained and used as a standard, i.e. it can be used over and over again for multiple different subjects. In other words, for example, when comparing a subject sample to a control sample, the data from the control sample could have been obtained in a different set of experiments, for example, it could be an average obtained from a number of healthy subjects and not actually obtained at the time the data for the subject was obtained.

The term "diagnosis" as used herein refers to methods by which the skilled artisan can estimate and/or determine whether or not a patient is suffering from a given disease or condition. The skilled artisan often makes a diagnosis on the basis of one or more diagnostic indicators, e.g., a marker, the presence, absence, amount, or change in amount of which is indicative of the presence, severity, or absence of the condition.

The term "prognosis" as used herein refers to is used herein to refer to the likelihood of a disease or condition progression, including recurrence of a disease or condition.

### Description of Methods of the Invention

Circulating disease cells are generally rare and often are apoptotic. Therefore, contaminating cells from the host/patient can overwhelm the signal from circulating disease cells in disease prognosis or diagnosis.

The present disclosure provides methods for diagnosing or aiding in the diagnosis of a disease or condition by comparing profiles (e.g., gene/protein/lipid/carbohydrate expression profiles, genotypes, gene copy number, gene dosage, DNA methylation, etc.) of disease or condition-associated markers (e.g., nucleic acids, proteins, lipids, carbohydrates, metabolites) between circulating diseased cells (e.g., cells affected by a disease or condition) and controls cells (e.g., circulating/or non-circulating normal cells, or circulating/or non-circulating cells that are substantially free of cells affected by the disease or condition) isolated from the same individual. Subtraction of the appropriate control cell(s) from the same individual can enrich the signal of disease-associated markers/analytes and enable the detection assay to achieve a high specificity, sensitivity, and accuracy. In some embodiments, such subtraction can further improve signal/noise ratio of the detection assay and/or improve the specificity, sensitivity, and accuracy of the detection assay.

The present disclosure also provides methods for diagnosing or aiding in the diagnosis of a disease or condition by comparing profiles (e.g., gene/protein/lipid/carbohydrate expression profiles, genotypes, gene copy number, gene dosage, DNA methylation, etc.) of disease or condition-associated markers (e.g., nucleic acids, proteins, lipids, carbohydrates, metabolites) between circulating diseased cells (e.g., cells affected by a disease or condition) and a control bodily fluid sample substantially free of cells affected by the disease or condition (e.g., a cell-free bodily fluid sample) isolated from the same individual. Subtraction of the appropriate control bodily fluid sample from the same individual can enrich the signal of disease-associated markers/analytes and enable the detection assay to achieve a high specificity, sensitivity, and accuracy. In some embodiments, such subtraction can further improve signal/noise ratio of the detection assay and/or improve the specificity, sensitivity, and accuracy of the detection assay.

This disclosure also provides methods for assessing the risk of developing a disease or condition, prognosing said disease, monitoring said disease progression or regression, assessing the efficacy of a treatment, or identifying a compound capable of ameliorating or treating said disease or condition.

Such a subject-specific profile comparison eliminates the dependence on a population-derived average profile for a particular disease or condition, which may introduce error into the detection or diagnosis of a particular disease or condition in the subject. The methods of this invention allow detection, diagnosis, and treatment to be personalized to the individual.

The methods of this invention (i) have high specificity, sensitivity, and accuracy and are capable of detecting disease or condition-specific markers present within a bodily fluid sample, cells or tissues; and (ii) eliminate the "inequality of baseline" that is known to occur among individuals due to intrinsic (e.g., age, gender, ethnic background, health status and the like) and temporal variations in marker expression. Accordingly, in certain aspects, the invention provides non-invasive assays for the early detection of a disease or condition, i.e., before the disease can be diagnosed by conventional diagnostic techniques, e.g., imaging techniques, and, therefore, provide a foundation for improved decision-making relative to the needs and strategies for intervention, prevention, and treatment of individuals with such disease or condition. The methods of this invention have improved specificity, sensitivity, and accuracy compared to current methods.

The methods of this invention can be used together with any known diagnostic methods, such as physical inspection, visual inspection, biopsy, scanning, histology, radiology, imaging, ultrasound, use of a commercial kit, genetic testing, immunological testing, analysis of bodily fluids, or monitoring neural activity.

Circulating diseased cells that can be used in the methods of this disclosure include all types of circulating cells that may be affected by a disease or condition or infected by an infectious agent. A circulating cell refers to a cell present in the bodily fluid. A circulating cell may not necessarily circulate throughout the entire body or in the circulatory system. For example, a circulating cell may be present locally, such as in synovial fluid, or cerebrospinal fluid, or lymph fluid. A circulating diseased cell may also be detached from a tissue or organ that has been affected by a disease or condition or infected by an infectious agent. In the invention the circulated diseased cells are blood cells.

Cells that can be used as control cells in the methods of this invention includes all types of normal cells, or healthy cells, or cells that are substantially free of a disease or condition, or cells that are substantially free of an infectious agent. Control cells may be circulating cells or non-circulating cells (e.g., a biopsied cell sample) that are representative of a normal or non-diseased state to which measurements on circulating diseased cells are compared to determine whether one or more diseased-associated marker is present in different levels between the circulating diseased cells and the control cells. The nature of the control cell may be a matter of design choice for a particular assay and may be derived or determined from normal tissue of the subject him- or herself.

A control bodily fluid sample that can be used in the methods of this invention includes all types of bodily fluids that are substantially free of cells that are affected by a disease or condition or that are substantially free of cell-free analytes associated with a disease or condition (e.g., cell-free nucleic acids). For example, the control bodily fluid sample may be isolated from a bodily fluid, such as blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, buccal swab sample, sputum, bronchial lavage, Pap smear sample, or ocular fluid. In some embodiments, the control bodily fluid sample is a cell-free bodily fluid, e.g., plasma or serum.

In certain embodiments, the control bodily fluid sample and the circulating diseased cells may be isolated from the same bodily fluid sample. For example, a populating of circulating tumor cells may be isolated from a whole blood sample and used to determine an analytical profile, while the remaining portion of the whole blood, or the remaining plasma or serum portion of the whole blood may be used as the control bodily fluid and to determine a control profile.

In the context of this disclosure, "control cells that are substantially free of cells affected by the disease or condition" refers to a population of cells, as compared to circulating diseased cells, comprise significantly fewer amounts of cells affected by the disease or condition. In some embodiments, "control cells that are substantially free of cells affected by the disease or condition" are cells that are at least 75%, 80%, 85%, 90%, 91%, 92%, 93%, 94%, 95%, 96%, 97%, 98%, 99%, 99.5%, or 100% free of cells affected by the disease or condition. In some embodiments, control cells that can be used in the methods of this invention are substantially free of fetal material (e.g., nucleic acids, proteins, and any analyte described herein), such as control cells that are at least 75%, 80%, 85%, 90%, 91%, 92%, 93%, 94%, 95%, 96%, 97%, 98%, 99%, 99.5%, or 100% free of fetal material. In the invention the control cells or the control bodily fluid sample are at least 75% free of cells affected by the disease or condition.

In the context of this disclosure, "a control bodily fluid that is substantially free of cells affected by the disease or condition" refers to a bodily fluid, as compared to circulating diseased cells, comprises significantly fewer amounts of cells affected by the disease or condition. In some embodiments, "a control bodily fluid that is substantially free of cells affected by the disease or condition" is a bodily fluid that is at least 75%, 80%, 85%, 90%, 91%, 92%, 93%, 94%, 95%, 96%, 97%, 98%, 99%, 99.5%, or 100% free of cells affected by the disease or condition. In some embodiments, a control bodily fluid is a cell-free bodily fluid (e.g., plasma or serum).

The circulating diseased cell that can be used in the methods of this disclosure may be present in any type of bodily fluid. For example, the bodily fluid sample may be blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, buccal swab sample, sputum, bronchial lavage, Pap smear sample, or ocular fluid. In the invention the circulating diseased cells are blood cells.

In the context of this disclosure, "a control bodily fluid free of cell-free analytes associated with a disease or condition" refers to a bodily fluid, as compared to circulating diseased cells, comprises significantly fewer amounts of analytes associated with the disease or condition (e.g., cell-free fetal DNA). In some embodiments, "a control bodily fluid free of cell-free analytes associated with a disease or condition" is a bodily fluid that is at least 75%, 80%, 85%, 90%, 91%, 92%, 93%, 94%, 95%, 96%, 97%, 98%, 99%, 99.5%, or 100% cell-free analytes associated with a disease or condition.

In some disclosed embodiments, the circulating diseased cells are blood cells, tumor cells, lymphoma cells, fetal cells, apoptotic cells, epithelia cells, endothelial cells, stem cells, progenitor cells, mesenchymal cells, osteoblast cells, osteocytes, hematopoietic stem cells, foam cells, adipose cells, transcervical cells, circulating cardiocytes, circulating fibrocytes, circulating cancer stem cells, circulating myocytes, circulating cells from kidney, circulating cells from gastrointestinal tract, circulating cells from lung, circulating cells from reproductive organs, circulating cells from central nervous system, circulating hepatic cells, circulating cells from spleen, circulating cells from thymus, circulating cells from thyroid , circulating cells from an endocrine gland, circulating cells from parathyroid, circulating cells from pituitary, circulating cells from adrenal gland, circulating cells from islets of Langerhans, circulating cells from pancreas, circulating cells from hypothalamus, circulating cells from prostate tissues, circulating cells from breast tissues, circulating cells from circulating retinal cells, circulating ophthalmic cells, circulating auditory cells, circulating epidermal cells, or circulating cells from the urinary tract. In other embodiments, the circulating diseased cells can be a mixture of different types of circulating diseased. In the invention however the circulating diseased cells are blood cells.

The circulating diseased cells that can be used in the methods of this invention may be affected by various diseases or conditions. Exemplary diseases or conditions are a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a pediatric disease, disorder, or condition, a mitochondrial disorder, a respiratory-gastrointestinal tract disease or condition, a reproductive disease or condition, an ophthalmic disease or condition, a musculo-skeletal disease or condition, or a dermal disease or condition.

The circulating diseased cells that can be used in the methods of this invention may be infected by an infectious agent, such as a virus, bacteria, fungus, parasite, protozoan, infectious protein, or microorganism.

In some embodiments, circulating diseased cells or control cells that may be used in the methods of this disclosure may be enucleated, for example, by using physical removal (e.g., via microneedle, optical tweezers, or aspiration), chemical treatments, photoablation, or ultraviolet irradiation.

As used herein, viruses include, but are not limited to, DNA or RNA animal viruses. As used herein, RNA viruses include, but are not limited to, virus families such as Picornaviridae (e.g., polioviruses), Reoviridae (e.g., rotaviruses), Togaviridae (e.g., encephalitis viruses, yellow fever virus, rubella virus), Orthomyxoviridae (e.g., influenza viruses), Paramyxoviridae (e.g., respiratory syncytial virus, measles virus, mumps virus, parainfluenza virus), Rhabdoviridae (e.g., rabies virus), Coronaviridae, Bunyaviridae, Flaviviridae, Filoviridae, Arenaviridae, Bunyaviridae and Retroviridae (e.g., human T cell lymphotropic viruses (HTLV), human immunodeficiency viruses (HIV)). As used herein, DNA viruses include, but are not limited to, virus families such as Papovaviridae (e.g., papilloma viruses), Adenoviridae (e.g., adenovirus), Herpesviridae (e.g., herpes simplex viruses), and Poxviridae (e.g., variola viruses).

As used herein, bacteria include, but are not limited to, gram positive bacteria, gram negative bacteria, acid-fast bacteria and the like. Gram positive bacteria include, but are not limited to, Actinomedurae, Actinomyces israelii, Bacillus anthracis, Bacillus cereus, Clostridium botulinum, Clostridium difficile, Clostridium perfringens, Clostridium tetani, Corynebacterium, Enterococcus faecalis, Listeria monocytogenes, Nocardia, Propionibacterium acnes, Staphylococcus aureus, Staphylococcus epiderm, Streptococcus mutans, Streptococcus pneumoniae and the like. Gram negative bacteria include, but are not limited to, Afipia felis, Bacteriodes, Bartonella bacilliformis, Bortadella pertussis, Borrelia burgdorferi, Borrelia recurrentis, Brucella, Calymmatobacterium granulomatis, Campylobacter, Escherichia coli, Francisella tularensis, Gardnerella vaginalis, Haemophilius aegyptius, Haemophilius ducreyi, Haemophilius influenziae, Heliobacter pylori, Legionella pneumophila, Leptospira interrogans, Neisseria meningitidia, Porphyromonas gingivalis, Providencia sturti, Pseudomonas aeruginosa, Salmonella enteridis, Salmonella typhi, Serratia marcescens, Shigella boydii, Streptobacillus moniliformis, Streptococcus pyogenes, Treponema pallidum, Vibrio cholerae, Yersinia enterocolitica, Yersinia pestis and the like. As used herein, acid-fast bacteria include, but are not limited to, Myobacterium avium, Myobacterium leprae, Myobacterium tuberculosis and the like. Other bacteria not falling into the other three categories include, but are not limited to, Bartonella henseiae, Chlamydia psittaci, Chlamydia trachomatis, Coxiella burnetii, Mycoplasma pneumoniae, Rickettsia akari, Rickettsia prowazekii, Rickettsia rickettsii, Rickettsia tsutsugamushi, Rickettsia typhi, Ureaplasma urealyticum, Diplococcus pneumoniae, Ehrlichia chafensis, Enterococcus faecium, Meningococci and the like.

As used herein, fungi include, but are not limited to, Aspergilli, Candidae, Candida albicans, Coccidioides immitis, Cryptococci, and combinations thereof.

As used herein, parasitic microbes include, but are not limited to, Balantidium coli, Cryptosporidium parvum, Cyclospora cayatanensis, Encephalitozoa, Entamoeba histolytica, Enterocytozoon bieneusi, Giardia lamblia, Leishmaniae, Plasmodii, Toxoplasma gondii, Trypanosomae, trapezoidal amoeba and the like. Other parasites include worms (e.g., helminthes), particularly parasitic worms including, but not limited to, Nematoda (roundworms, e.g., whipworms, hookworms, pinworms, ascarids, filarids and the like), and Cestoda (e.g., tapeworms).

As used herein, infectious proteins include prions (e.g., PrP^{Sc} forms, the CJD prion, the vCJD prion, the FFI prion, and the GSS prion).

As used herein, the term "a pediatric disease, disorder, or condition" refers to any disease, disorder, or condition that affects infants, or children or that begins during development or childhood. Examples of pediatric diseases, disorders, and conditions include, but are not limited to, autism, Kawasaki's disease, congenital deafness, pediatric cancers, Type I diabetes, congenital heart defects, tetralogy of Fallot, Duchenne Muscular Dystrophy, osteogenesis importfect, Krabe disease, Pompe disease, Gaucher disease, Fabry disease, Wolff-Parkinson-White syndrome, Hirschsprung's disease, Crohn's disease, Eagle-Barrett Syndrome, cystic fibrosis, irritable bowel syndrome, and cerebral palsy. Examples of pediatric conditions also include genetic attributes of the developing fetus. For example, pediatric conditions include, but are not limited to, intelligence, eye color, hair color, and muscle type.

According to the present invention, disease-specific markers (e.g., nucleic acids, proteins, carbohydrates and/or lipids) may be expressed or present in the circulating diseased cells that can be used in the methods of the present invention.

As used herein, a "profile" of a marker of a disease or condition can broadly refer to any information concerning the marker. This information can be either qualitative (e.g., presence or absence) or quantitative (e.g., levels, copy numbers, or dosages). In some embodiments, a profile of a marker can indicate the absence of this marker. The profile can be a nucleic acid (e.g., DNA or RNA) profile, a protein profile, a lipid profile, a carbohydrate profile, a metabolite profile, or a combination thereof. A "marker" as used herein generally refers to an analyte which is differentially detectable in circulating diseased cells and is indicative of the presence of a disease or condition. An analyte is differentially detectable if it can be distinguished quantitatively or qualitatively between circulating diseased cells and control cells (or a control bodily fluid).

The methods of this invention can be applied to various diseases or conditions. Exemplary diseases or conditions are a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a pediatric disease, disorder, or condition, a mitochondrial disorder, a respiratory-gastrointestinal tract disease or condition, a reproductive disease or condition, an ophthalmic disease or condition, a musculo-skeletal disease or condition, or a dermal disease or condition.

As used herein, the term "cardiovascular disease or condition" refers to any condition that affects systems of heart or blood vessels (arteries and veins). Examples of cardiovascular diseases include, but are not limited to myocardial infarction, coronary artery disease, percutaneous transluminal coronary angioplasty (PTCA), coronary artery bypass surgery (CABG), restenosis, peripheral arterial disease, stroke, abdominal aorta aneurysm, intracranial aneurysm, large artery atherosclerotic stroke, cardiogenic stroke, an early onset myocardial infarction, heart failure, pulmonary embolism, acute coronary syndrome (ACS), angina, cardiac hypertrophy, arteriosclerosis, myocarditis, pancarditis, endocarditis, hypertension, congestive heart failure, atherosclerosis, cerebrovascular disease, declining cardiac health, ischemic heart disease, pericarditis, cardiogenic shock, alcoholic cardiomyopathy, congenital heart disease, ischemic cardiomyopathy, hypertensive cardiomyopathy, valvular cardiomyopathy, inflammatory cardiomyopathy, cardiomyopathy secondary to a systemic metabolic disease, dilated cardiomyopathy, hypertrophic cardiomyopathy, arrhythmogenic right ventricular cardiomyopathy, restrictive cardiomyopathy, noncompaction cardiomyopathy, valvular heart disease, hypertensive heart disease, myocardial ischemic attack, unstable angina, myocardial rupture, cardiogenic shock, embolism, deep vein thrombosis, arrhythmia, arrhythmogenic right ventricular cardiomyopathy, diabetic cardiomyopathy, mitral regurgitation, mitral valve prolapse, peripheral vascular disease, artery disease, carotid artery disease, deep vein thrombosis, venous diseases, cerebrovascular disease, arterial aneurysm, left ventricular hypertrophy, hypertensive renal disease, hypertensive retinal disease, vasculitis, left main disease, arterial vascular disease, venous vascular disease, thrombosis of the microcirculation, a transient cerebrovascular accident, limb ischemia, aneurysm, thrombosis, superficial venous thrombosis, and deep venous thrombosis.

As used herein, the term "kidney-associated disease or condition" refers to any disease or condition that affects kidney or renal system. Examples of kidney-associated disease include, but are not limited to, chronic kidney diseases, primary kidney diseases, non-diabetic kidney diseases, glomerulonephritis, interstitial nephritis, diabetic kidney diseases, diabetic nephropathy, glomerulosclerosis, rapid progressive glomerulonephritis, renal fibrosis, Alport syndrome, IDDM nephritis, mesangial proliferative glomerulonephritis, membrano proliferative glomerulonephritis, crescentic glomerulonephritis, renal insterstitial fibrosis, focal segmental glomerulosclerosis, membranous nephropathy, minimal change disease, pauci-immune rapid progressive glomerulonephritis, IgA nephropathy, polycystic kidney disease, Dent's disease, nephrocytinosis, Heymann nephritis, autosomal dominant (adult) polycystic kidney disease, autosomal recessive (childhood) polycystic kidney disease, acute kidney injury, nephrotic syndrome, renal ischemia, podocyte diseases or disorders, proteinuria, glomerular diseases, membranous glomerulonephritis, focal segmental glomerulonephritis, pre-eclampsia, eclampsia, kidney lesions, collagen vascular diseases, benign orthostatic (postural) proteinuria, IgM nephropathy, membranous nephropathy, sarcoidosis, diabetes mellitus, kidney damage due to drugs, Fabry's disease, aminoaciduria, Fanconi syndrome, hypertensive nephrosclerosis, interstitial nephritis, Sickle cell disease, hemoglobinuria, myoglobinuria, Wegener's Granulomatosis, Glycogen Storage Disease Type 1, chronic kidney disease, chronic renal failure, low Glomerular Filtration Rate (GFR), nephroangiosclerosis, lupus nephritis, ANCA-positive pauci-immune crescentic glomerulonephritis, chronic allograft nephropathy, nephrotoxicity, renal toxicity, kidney necrosis, kidney damage, glomerular and tubular injury, kidney dysfunction, nephritic syndrome, acute renal failure, chronic renal failure, proximal tubal dysfunction, acute kidney transplant rejection, chronic kidney transplant refection, non IgA mesangioproliferative glomerulonephritis, postinfectious glomerulonephritis, vasculitides with renal involvement of any kind, any hereditary renal disease, any interstitial nephritis, renal transplant failure, kidney cancer, kidney disease associated with other conditions (e.g., hypertension, diabetes, and autoimmune disease), Dent's disease, nephrocytinosis, Heymann nephritis, a primary kidney disease, a collapsing glomerulopathy, a dense deposit disease, a cryoglobulinemia-associated glomerulonephritis, an Henoch-Schonlein disease, a postinfectious glomerulonephritis, a bacterial endocarditis, a microscopic polyangitis, a Churg-Strauss syndrome, an anti-GBM-antibidy mediated glomerulonephritis, amyloidosis, a monoclonal immunoglobulin deposition disease, a fibrillary glomerulonephritis, an immunotactoid glomerulopathy, ischemic tubular injury, a medication-induced tubulo-interstitial nephritis, a toxic tubulo-interstitial nephritis, an infectious tubulo-interstitial nephritis, a bacterial pyelonephritis, a viral infectious tubulo-interstitial nephritis which results from a polyomavirus infection or an HIV infection, a metabolic-induced tubulo-interstitial disease, a mixed connective disease, a cast nephropathy, a crystal nephropathy which may results from urate or oxalate or drug-induced crystal deposition, an acute cellular tubulo-interstitial allograft rejection, a tumoral infiltrative disease which results from a lymphoma or a post-transplant lymphoproliferative disease, an obstructive disease of the kidney, vascular disease, a thrombotic microangiopathy, a nephroangiosclerosis, an atheroembolic disease, a mixed connective tissue disease, a polyarteritis nodosa, a calcineurin-inhibitor induced- vascular disease, an acute cellular vascular allograft rejection, an acute humoral allograft rejection, early renal function decline (ERFD), end stage renal disease (ESRD), renal vein thrombosis, acute tubular necrosis, acute interstitial nephritis, established chronic kidney disease, renal artery stenosis, ischemic nephropathy, uremia, drug and toxin-induced chronic tubulointerstitial nephritis, reflux nephropathy, kidney stones, Goodpasture's syndrome, and hydronephrosis.

As used herein, the term "prenatal or pregnancy-related disease or condition" refers to any disease, disorder, or condition affecting a pregnant woman, embryo, or fetus. Prenatal or pregancy-related conditions can also refer to any disease, disorder, or condition that is associated with or arises, either directly or indirectly, as a result of pregnancy. These diseases or conditions can include any and all birth defects, congenital conditions, or hereditary diseases or conditions. Examples of prenatal or pregnancy-related diseases include, but are not limited to, Rhesus disease, hemolytic disease of the newborn, beta-thalassemia, sex determination, determination of pregnancy, a hereditary Mendelian genetic disorder, chromosomal aberrations, a fetal chromosomal aneuploidy, fetal chromosomal trisomy, fetal chromosomal monosomy, trisomy 8, trisomy 13 (Patau Syndrom), trisomy 16, trisomy 18 (Edwards syndrome), trisomy 21 (Down syndrome), X-chromosome linked disorders, trisomy X (XXX syndrome), monosomy X (Turner syndrome), XXY syndrome, XYY syndrome, XYY syndrome, XXXY syndrome, XXYY syndrome, XYYY syndrome, XXXXX syndrome, XXXXY syndrome, XXXYY syndrome, XXYYY syndrome, Fragile X Syndrome, fetal growth restriction, cystic fibrosis, a hemoglobinopathy, fetal death, fetal alcohol syndrome, sickle cell anemia, hemophilia, Klinefelter syndrome, dup(17)(p11.2p1.2) syndrome, endometriosis, Pelizaeus-Merzbacher disease, dup(22)(q11.2q11.2) syndrome, cat eye syndrome, cri-du-chat syndrome, Wolf-Hirschhorn syndrome, Williams-Beuren syndrome, Charcot-Marie-Tooth disease, neuropathy with liability to pressure palsies, Smith-Magenis syndrome, neurofibromatosis, Alagille syndrome, Velocardiofacial syndrome, DiGeorge syndrome, steroid sulfatase deficiency, Prader-Willi syndrome, Kallmann syndrome, microphthalmia with linear skin defects, adrenal hypoplasia, glycerol kinase deficiency, Pelizaeus-Merzbacher disease, testis-determining factor on Y, azospermia (factor a), azospermia (factor b), azospermia (factor c), 1p36 deletion, phenylketonuria, Tay-Sachs disease, adrenal hyperplasia, Fanconi anemia, spinal muscular atrophy, Duchenne's muscular dystrophy, Huntington's disease, myotonic dystrophy, Robertsonian translocation, Angelman syndrome, tuberous sclerosis, ataxia telangieltasia, open spina bifida, neural tube defects, ventral wall defects, small-for-gestational-age, congenital cytomegalovirus, achondroplasia, Marfan's syndrome, congenital hypothyroidism, congenital toxoplasmosis, biotinidase deficiency, galactosemia, maple syrup urine disease, homocystinuria, medium-chain acyl Co-A dehydrogenase deficiency, structural birth defects, heart defects, abnormal limbs, club foot, anencephaly, arhinencephaly/holoprosencephaly, hydrocephaly, anophthalmos/microphthalmos, anotia/microtia, transposition of great vessels, tetralogy of Fallot, hypoplastic left heart syndrome, coarctation of aorta, cleft palate without cleft lip, cleft lip with or without cleft palate, oesophageal atresia/stenosis with or without fistula, small intestine atresia/stenosis, anorectal atresia/stenosis, hypospadias, indeterminate sex, renal agenesis, cystic kidney, preaxial polydactyly, limb reduction defects, diaphragmatic hernia, blindness, cataracts, visual problems, hearing loss, deafness, X-linked adrenoleukodystrophy, Rett syndrome, lysosomal disorders, cerebral palsy, autism, aglossia, albinism, ocular albinism, oculocutaneous albinism, gestational diabetes, Arnold-Chiari malformation, CHARGE syndrome, congenital diaphragmatic hernia, brachydactlia, aniridia, cleft foot and hand, heterochromia, Dwarnian ear, Ehlers Danlos syndrome, epidermolysis bullosa, Gorham's disease, Hashimoto's syndrome, hydrops fetalis, hypotonia, Klippel-Feil syndrome, muscular dystrophy, osteogenesis imperfecta, progeria, Smith Lemli Opitz symdrom, chromatelopsia, X-linked lymphoproliferative disease, omphalocele, gastroschisis, pre-eclampsia, eclampsia, pre-term labor, premature birth, miscarriage, delayed intrauterine growth, ectopic pregnancy, hyperemesis gravidarum, morning sickness, or likelihood for successful induction of labor.

As used herein, the term "a neurological or neuropsychiatric disease or condition" refers to any disease or condition that affects nervous systems. Examples of neurological or neuropsychiatric diseases or conditions include, but are not limited to, head trauma, stroke, stroke, ischemic stroke, hemorrhagic stroke, subarachnoid hemorrhage, intra cranial hemorrhage, transient ischemic attack, vascular dementia, corticobasal ganglionic degeneration, encephalitis, epilepsy, Landau-Kleffner syndrome, hydrocephalus, pseudotumor cerebri, thalamic diseases, meningitis, myelitis, movement disorders, essential tremor, spinal cord diseases, syringomyelia, Alzheimer's disease (early onset), Alzheimer's disease (late onset), multi-infarct dementia, Pick's disease, Huntingdon's disease, Parkinson's disease, Parkinson syndromes, dementia, frontotemporal dementia, corticobasal degeneration, multiple system atrophy, progressive supranuclear palsy, Lewy body disease, Creutzfeldt-Jakob disease, Dandy-Walker syndrome, Friedreich ataxia, Machado-Joseph disease, migraine, schizophrenia, mood disorders and depression, dementia with lewy bodies (DLB), frontotemporal dementia (FTD), various forms of vascular dementia (VD), subcortical vascular dementia (Binswanger's disease), autism, developmental retardations, motor neuron diseases, amyotrophic lateral sclerosis (ALS), neuronal or brain damage, hypoxia of the brain, cerebral palsy (CP), memory disorders, movement disorders, corticalbasal ganglionic degeneration, forms of multiple system atrophy, stroke-related disorders, cerebrovascular accidents, post-irradiation encephalopathy with seizures, vascular Parkinsonism, thalamic cerebrovascular accidents, chronic inflammatory demyelinating polyneuropathy, alcohol related dementia, semantic dementia, ataxia, atypical Parkinsonism, dystonia, progressive supranuclear palsy, essential tremor, mild cognitive impairment, amyotrophic lateral sclerosis, multiple sclerosis, neuropathies, Pick's disease, congophilic amyloid angiopathy, Creutzfeldt-Jakob Disease, AIDS dementia complex, depression, anxiety disorder, phobia, Bell's Palsy, epilepsy, encephalitis, neuromuscular disorders, neurooncological disorders, brain tumors, neurovascular disorders, neuroimmunological disorders, neurootological disease, neurotrauma including spinal cord injury, pain including neuropathic pain, pediatric neurological and neuropsychiatric disorders, sleep disorders, Tourette syndrome, corticalbasal ganglionic degeneration, alcohol related dementia, semantic dementia, Alzheimer's disease combined with multi-infarct dementia, Alzheimer's disease combined with Lewy body dementia, Parkinson's disease combined with Lewy body dementia, Alzheimer's and Parkinson's disease combined with Lewy body dementia, frontotemporal dementia combined with chronic inflammatory demyelinating polyneuropathy, attention deficit hyperactivity disorder, schizophrenia, obsessive-compulsive disorder, mental retardation, autistic spectrum disorders, opsoclonus-myoclonus syndrome (OMS) seizures, articulation disorder, learning disabilities (i.e., reading or arithmetic), verbal or performance aptitude deficits, attention deficit disorder, amyloid diseases, prion diseases, Tauopathies, Alpha-Synucleinopathies, addictive states such as those caused by at least one of: cocaine, nicotine, alcohol, food, ecstasy, kat, caffeine, opium, heroin, marijuana, amphetamine, methamphetamine or gambling, and Fabry's disease.

As used herein, the term "an autoimmune or immune-related disease or condition" refers to any disease or condition that affects the function of immune systems. Examples of autoimmune or immune-related diseases or conditions include, but are not limited to, antiphospholipid syndrome, systemic lupus erythematosus, rheumatoid arthritis, autoimmune vasculitis, celiac disease, autoimmune thyroiditis, post-transfusion immunization, maternal-fetal incompatibility, transfusion reactions, immunological deficiency such IgA deficiency, common variable immunodeficiency, drug-induced lupus, diabetes mellitus, Type I diabetes, Type II diabetes, juvenile onset diabetes, juvenile rheumatoid arthritis, psoriatic arthritis, multiple sclerosis, immunodeficiency, allergies, asthma, psoriasis, atopic dermatitis, allergic contact dermatitis, chronic skin diseases, amyotrophic lateral sclerosis, chemotherapy-induced injury, graft-vs-host diseases, bone marrow transplant rejection, Ankylosing spondylitis, atopic eczema, Pemphigus, Behcet's disease, chronic fatigue syndrome fibromyalgia, chemotherapy-induced injury, myasthenia gravis, glomerulonephritis, allergic retinitis, systemic sclerosis, subacute cutaneous lupus erythematosus, cutaneous lupus erythematosus including chilblain lupus erythematosus, Sjogren's syndrome, autoimmune nephritis, autoimmune vasculitis, autoimmune hepatitis, autoimmune carditis, autoimmune encephalitis, autoimmune mediated hematological diseases, lc-SSc (limited cutaneous form of scleroderma), dc-SSc (diffused cutaneous form of scleroderma), autoimmune thyroiditis (AT), Grave's disease (GD), myasthenia gravis, multiple sclerosis (MS), ankylosing spondylitis. transplant rejection, immune aging, rheumatic/autoimmune diseases, mixed connective tissue disease, spondyloarthropathy, psoriasis, psoriatic arthritis, myositis, scleroderma, dermatomyositis, autoimmune vasculitis, mixed connective tissue disease, idiopathic thrombocytopenic purpura, Crohn's disease, human adjuvant disease, osteoarthritis, juvenile chronic arthritis, a spondyloarthropathy, an idiopathic inflammatory myopathy, systemic vasculitis, sarcoidosis, autoimmune hemolytic anemia, autoimmune thrombocytopenia, thyroiditis, immune-mediated renal disease, a demyelinating disease of the central or peripheral nervous system, idiopathic demyelinating polyneuropathy, Guillain-Barre syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease, psoriatic arthritis, psoriasis, dermatitis, polymyositis/dermatomyositis, toxic epidermal necrolysis, systemic scleroderma and sclerosis, responses associated with inflammatory bowel disease, Crohn's disease, ulcerative colitis, respiratory distress syndrome, adult respiratory distress syndrome (ARDS), meningitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, allergic encephalomyelitis, immune responses associated with acute and delayed hypersensitivity mediated by cytokines and T-lymphocytes, tuberculosis, sarcoidosis, granulomatosis including Wegener's granulomatosis, agranulocytosis, vasculitis (including ANCA), aplastic anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, mysathenia gravis, antigen-antibody complex mediated diseases, anti-glomerular basement membrane disease, antiphospholipid antibody syndrome, allergic neuritis, Bechet disease, Castleman's syndrome, Goodpasture's syndrome, Lambert-Eaton Myasthenic Syndrome, Reynaud's syndrome, Sjorgen's syndrome, Stevens-Johnson syndrome, pemphigoid bullous, pemphigus, autoimmune polyendocrinopathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), autoimmune thrombocytopenia, autoimmune disease of the testis and ovary including autoimmune orchitis and oophoritis, primary hypothyroidism, autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Sheehan's syndrome, autoimmune hepatitis, lymphoid interstitial pneumonitis (HIV), bronchiolitis obliterans (non-transplant) vs NSIP, Guillain-Barre' Syndrome, large vessel vasculitis (including polymyalgia rheumatica and giant cell (Takayasu's) arteritis), medium vessel vasculitis (including Kawasaki's disease and polyarteritis nodosa), ankylosing spondylitis, Berger's disease (IgA nephropathy), rapidly progressive glomerulonephritis, primary biliary cirrhosis, Celiac sprue (gluten enteropathy), cryoglobulinemia, and amyotrophic lateral sclerosis (ALS).

As used herein, the term "cancer" refers to various types of malignant neoplasms, most of which can invade surrounding tissues, and may metastasize to different sites (see, for example, PDR Medical Dictionary, 1st edition (1995)). The terms "neoplasm" and "tumor" refer to an abnormal tissue that grows by cellular proliferation more rapidly than normal and continues to grow after the stimuli that initiated proliferation is removed. Such abnormal tissue shows partial or complete lack of structural organization and functional coordination with the normal tissue which may be either benign (i.e., benign tumor) or malignant (i.e., malignant tumor). Examples of general categories of cancer include, but are not limited to, carcinomas (i.e., malignant tumors derived from epithelial cells such as, for example, common forms of breast, prostate, lung and colon cancer), sarcomas (i.e., malignant tumors derived from connective tissue or mesenchymal cells), lymphomas (i.e., malignancies derived from hematopoietic cells), leukemias (i.e., malignancies derived from hematopoietic cells), germ cell tumors (i.e., tumors derived from totipotent cells. In adults most often found in the testicle or ovary; in fetuses, babies and young children, most often found on the body midline, particularly at the tip of the tailbone), blastic tumors (i.e., a typically malignant tumor which resembles an immature or embryonic tissue) and the like. Examples of the types of neoplasms intended to be encompassed by the present disclosure include but are not limited to those neoplasms associated with cancers of neural tissue, blood forming tissue, breast, skin, bone, prostate, ovaries, uterus, cervix, liver, lung, brain, larynx, gallbladder, pancreas, rectum, parathyroid, thyroid, adrenal gland, immune system, head and neck, colon, stomach, bronchi, and/or kidneys.

As used herein, the term "an infectious disease or condition" refers to any disease or condition that results from an infectious agent. Infectious agents include, but are not limited to bacteria, viruses, fungi, protozoa, infectious proteins, parasitic microbes, and other parasites. Examples of infectious diseases or conditions include, but are not limited to, bacterial infections, viral infections, fungal infections, protozoan infections, parasitic infections, hepatitis (e.g., hepatitis A, B, C, D, and E), herpes, influenza, human papillomavirus (HPV) infection, AIDS, anthrax, pneumonia (bacterial or viral), cellulitis, human parainfluenza, the common cold, Legionnaires' disease (Legionellosis), cholera, Creutzfeldt-Jakob disease (CJD), variant Creutzfeldt-Jakob disease (vCJD), fatal familial insomnia (FFI), Gerstmann-Sträussler-Scheinker (GSS) syndrome, Chlamydia, chicken pox, ebola hemorrhagic fever, Dengue fever, giardiasis, Lyme disease, malaria, measles, mumps, rubella, pertussis, gonorrhea, staphylococcal infection, streptococcal infection, pneumococcal infection, rabies, helicobacter pylori infection, respiratory syncitial virus infection, Rocky Mountain spotted fever, SARS, sepsis, tuberculosis, and West Nile fever.

As used herein, "treating" a disease or condition refers to taking steps to obtain beneficial or desired results, including clinical results. Beneficial or desired clinical results include, but are not limited to, alleviation or amelioration of one or more symptoms associated with diseases or conditions.

As used herein, "administering" or "administration of' a compound or an agent to a subject can be carried out using one of a variety of methods known to those skilled in the art. For example, a compound or an agent can be administered, intravenously, arterially, intradermally, intramuscularly, intraperitonealy, intravenously, subcutaneously, ocularly, sublingually, orally (by ingestion), intranasally (by inhalation), intraspinally, intracerebrally, and transdermally (by absorbtion, e.g., through a skin duct). A compound or agent can also appropriately be introduced by rechargeable or biodegradable polymeric devices or other devices, e.g., patches and pumps, or formulations, which provide for the extended, slow, or controlled release of the compound or agent. Administering can also be performed, for example, once, a plurality of times, and/or over one or more extended periods. In some aspects, the administration includes both direct administration, including self-administration, and indirect administration, including the act of prescribing a drug. For example, as used herein, a physician who instructs a patient to self-administer a drug, or to have the drug administered by another and/or who provides a patient with a prescription for a drug is administering the drug to the patient. In some embodiments, a compound or an agent is administered orally, e.g., to a subject by ingestion, or intravenously, e.g., to a subject by injection. In some embodiments, the orally administered compound or agent is in an extended release or slow release formulation, or administered using a device for such slow or extended release.

In certain embodiments, markers used in the methods of invention are up-regulated or activated in the circulating diseased cells compared to the control cells or the control bodily fluid sample. In certain embodiments, markers used in the methods of invention are down-regulated or inhibited in the circulating diseased cells compared to the control cells or the control bodily fluid sample. Different diseases or conditions can be associated with either up-regulation (or activation) or down-regulation (or inhibition) of different markers. As used herein, "up-regulation or up-regulated" can refer to an increase in expression levels (e.g., gene expression or protein expression), gene copy numbers, gene dosages, and other qualitative or quantitative detectable state of the markers. Similarly, "down-regulation or down-regulated" can refer to an increase in expression levels, gene copy numbers, gene dosages, and other qualitative or quantitative detectable state of the markers. As used herein, "activation or activated" can refer to an active state of the marker, e.g., a phosphorylation state, a DNA methylation state, or a DNA acetylation state. Similarly, "inhibition or inhibited" can refer to a repressed state or an inactivated state of the marker, e.g., a de-phosphorylation state, a ubiquitination state, a DNA de-methylation state.

In certain embodiments, methods of this disclosure also comprise at least one of the following steps before determination of various profiles: i) lysing the circulating diseased cells and the control cells; and ii) extracting cellular contents from the lysed circulating diseased cells and the lysed control cells. In certain embodiments, at least one or more markers of a disease or condition are present in the cellular contents of the circulating diseased cells. In certain embodiments, there is no marker present in the cellular contents of the control cells or the control bodily fluid sample. In certain embodiments, methods of this disclosure can comprise extracting or enriching markers/analytes from circulating diseased cells. Any known extraction and enrichment methods can be used herein.

In certain embodiments, methods of this disclosure further comprise comparing the identified difference of the disease or condition-specific markers to a repository of at least one markers known in the art. Such comparison can further confirm the presence of the disease or condition. In some embodiments, the repository of the known markers can be obtained by data mining. The term "data mining", as used herein, refers to a process of finding new data patterns, relations, or correlations derived from the known data of the databases and of extracting practicable information in the future. Typically a computer-based system can be trained on data to perform the data mining, e.g., to classify the input data and then subsequently used with new input data to make decisions based on the training data. These systems include, but are not limited, expert systems, fuzzy logic, nonlinear regression analysis, multivariate analysis, decision tree classifiers, and Bayesian belief networks.

In the methods of this disclosure, cell separation/isolation/purification methods are used to isolate populations of circulating diseased cells from a bodily fluid sample, cells, or tissues of a subject. Circulating diseased cells may be rare or in low quantity in a bodily fluid. Therefore, enrichment techniques (e.g., magnetic enrichment) may be used to enrich circulating diseased cells before the isolation or before determining the profile. A skilled worker can use any known cell separation/isolation/purification techniques to isolate circulating diseased cells from a bodily fluid containing circulating diseased cells. Exemplary techniques include, but are not limited to, using antibodies, flow cytometry, fluorescence activated cell sorting, filtration, microchip techniques, ultracentrifugation, antibody isolation, chromatographic isolation, gradient-based centrifugation, elution, microfluidics, magnetic separation technique, fluorescent-magnetic separation technique, nanostructure, quantum dots, high throughput microscope-based platform, micro-fluidic technique, fiber-optic array-scanning technique, laser-scanning cytometry technique, multiphoton intravital flow cytometry, photoacoustic flowmetry, nanoparticles targeting cell surface antigens, staining circulating diseased cells with detectable secreted products, or a combination thereof. Circulating diseased cells may have different physical properties compared to normal circulating cells, such as difference in size, density, charge, migratory properties, and some properties of specific cell types (e.g., melanocytic granules in circulating melanoma cells). A skilled worker can use any known cell separation/isolation/purification techniques based on such different properties to isolate circulating diseased cells. For example, differences in buoyant density may be used to separate circulating diseased cells (e.g., circulating tumor cells) from normal blood cells through gradient centrifugation. Filtration-based approaches may be used isolate circulating diseased cells (e.g., circulating tumor cells) based on their increased sizes compared to normal circulating cells. Antibody-based isolation approaches may be used to capture circulating diseased cells, which express epithelia cell surface markers that are absent from normal circulating blood cells. For example, conjugation of antibodies against epithelial cell adhesion molecule (EpCAM) to magnetic beads, followed by purification of captured cells through a magnetic field, may be used to enrich circulating tumor cells from the blood of patients with cancers of the breast, prostate, and colon. In certain embodiments, circulating diseased cells (e.g., transcervical cells) may be collected by the RareCellect™ device (Genetic Technologies) or similar devices.

In certain embodiments, the circulating diseased cells are isolated by using a product secreted by the circulating diseased cells. In certain embodiments, the circulating diseased cells are isolated by using a cell surface target (e.g., receptor protein) on the surface of the circulating diseased cells. In some embodiments, the cell surface target is expressed by the circulating diseased cells on their plasma membranes. In some embodiments, the cell surface target is expressed by the circulating diseased cells on their plasma membranes. In some embodiments, the cell surface target is an exogenous protein that is translocated on the plasma membranes, but not expressed by the circulating diseased cells. In some embodiments, the cell surface target is a marker of the disease or condition to be detected.

In certain aspects of the methods described herein, analytes include nucleic acids, proteins, lipids, carbohydrates, metabolites, or any combinations of these. In certain aspects of the methods described herein, markers include nucleic acids, proteins, lipids, carbohydrates, metabolites, or any combinations of these. As used herein, the term "nucleic acid" is intended to include DNA molecules (e.g., cDNA or genomic DNA), RNA molecules (e.g., mRNA), DNA-RNA hybrids, and analogs of the DNA or RNA generated using nucleotide analogs. The nucleic acid molecule can be a nucleotide, oligonucleotide, double-stranded DNA, single-stranded DNA, multi-stranded DNA, complementary DNA, genomic DNA, non-coding DNA, messenger RNA (mRNAs), microRNA (miRNAs), small nucleolar RNA (snoRNAs), ribosomal RNA (rRNA), transfer RNA (tRNA), small interfering RNA (siRNA), heterogeneous nuclear RNAs (hnRNA), or small hairpin RNA (shRNA).

As used herein, the term "amino acid" includes organic compounds containing both a basic amino group and an acidic carboxyl group. Included within this term are natural amino acids (e.g., L-amino acids), modified and unusual amino acids (e.g., D-amino acids and β-amino acids), as well as amino acids which are known to occur biologically in free or combined form but usually do not occur in proteins. Natural protein occurring amino acids include alanine, arginine, asparagine, aspartic acid, cysteine, glutamic acid, glutamine, glycine, histidine, isoleucine, leucine, lysine, methionine, phenylalanine, serine, threonine, tyrosine, tryptophan, proline, and valine. Natural non-protein amino acids include arginosuccinic acid, citrulline, cysteine sulfuric acid, 3,4-dihydroxyphenylalanine, homocysteine, homoserine, ornithine, 3-monoiodotyrosine, 3,5-diiodotryosine, 3, 5, 5-triiodothyronine, and 3,3',5,5'- tetraiodothyronine. Modified or unusual amino acids include D-amino acids, hydroxylysine, 4-hydroxyproline, N-Cbz-protected amino acids, 2,4-diaminobutyric acid, homoarginine, norleucine, N-methylaminobutyric acid, naphthylalanine, phenylglycine, .alpha.-phenylproline, tert-leucine, 4-aminocyclohexylalanine, N-methyl-norleucine, 3 ,4-dehydroproline, N,N-dimethylaminoglycine, N-methylaminoglycine, 4-aminopiperidine-4-carboxylic acid, 6-aminocaproic acid, trans-4-(aminomethyl)-cyclohexanecarboxylic acid, 2-, 3-, and 4-(aminomethyl)- benzoic acid, 1-aminocyclopentanecarboxylic acid, 1-aminocyclopropanecarboxylic acid, and 2-benzyl-5-aminopentanoic acid.

As used herein, the term "peptide" includes compounds that consist of two or more amino acids that are linked by means of a peptide bond. Peptides may have a molecular weight of less than 10,000 Daltons, less than 5,000 Daltons, or less than 2,500 Daltons. The term "peptide" also includes compounds containing both peptide and non-peptide components, such as pseudopeptide or peptidomimetic residues or other non-amino acid components. Such compounds containing both peptide and non-peptide components may also be referred to as a "peptide analog."

As used herein, the term "protein" includes compounds that consist of amino acids arranged in a linear chain and joined together by peptide bonds between the carboxyl and amino groups of adjacent amino acid residues. Proteins used in methods of the invention include, but are not limited to, amino acids, peptides, antibodies, antibody fragments, cytokines, lipoproteins, or glycoproteins.

As used herein, the term "antibody" includes polyclonal antibodies, monoclonal antibodies (including full length antibodies which have an immunoglobulin Fc region), antibody compositions with polyepitopic specificity, multispecific antibodies (e.g., bispecific antibodies, diabodies, and single-chain molecules, and antibody fragments (e.g., Fab or F(ab')₂, and Fv). For the structure and properties of the different classes of antibodies, see e.g., Basic and Clinical Immunology, 8th Edition, Daniel P. Sties, Abba I. Terr and Tristram G. Parsolw (eds), Appleton & Lange, Norwalk, Conn., 1994, page 71 and Chapter 6.

As used herein, the term "cytokine" refers to a secreted protein or active fragment or mutant thereof that modulates the activity of cells of the immune system. Examples of cytokines include, without limitation, interleukins, interferons, chemokines, tumor necrosis factors, colony-stimulating factors for immune cell precursors, and the like.

As used herein, the term "lipoprotein" includes negatively charged compositions that comprise a core of hydrophobic cholesteryl esters and triglyceride surrounded by a surface layer of amphipathic phospholipids with which free cholesterol and apolipoproteins are associated. Lipoproteins may be characterized by their density (e.g. very-low-density lipoprotein (VLDL), low-density lipoprotein (LDL) and high density lipoprotein (HDL)), which is determined by their size, the relative amounts of lipid and protein. Lipoproteins may also be characterized by the presence or absence of particular modifications (e.g. oxidization, acetylation, or glycation).

As used herein, the term "glycoprotein" includes glycosides which have one or more oligo- or polysaccharides covalently attached to a peptide or protein. Exemplary glycoproteins can include, without limitation, immunoglobulins, members of the major histocompatibility complex, collagens, mucins, glycoprotein IIb/IIIa, glycoprotein-41 (gp41) and glycoprotein-120 (gp12), follicle-stimulating hormone, alpha-fetoprotein, erythropoietin, transferrins, alkaline phosphatase, and lectins.

As used herein, the term "lipid" includes synthetic or naturally-occurring compounds which are generally amphipathic and biocompatible. Lipids typically comprise a hydrophilic component and a hydrophobic component. Exemplary lipids include, but are not limited to fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, sulfatide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lyso-sphingomyelin,, gangliosides, plasmalogen, sulfatide, ceramide, low density lipoproteins (LDLs), very low density lipoproteins (VLDLs), high density lipoproteins (HDLs), sphingoid base-1-phosphates or derivatives thereof.

As used herein, the term "carbohydrate" includes, but is not limited to, compounds that contain oxygen, hydrogen and carbon atoms, typically (CH₂O)ₙ wherein n is an integer. Exemplary carbohydrates include, but are not limited to, monosaccharides, disaccharides, polysaccharides, or oligosaccharides.

As used herein, the term "metabolite" includes any molecule used in metabolism. Metabolites can be products, substrates, or intermediates in metabolic processes. Included within this term are primary metabolites, secondary metabolites, organic metabolites, or inorganic metabolites. Metabolites include, without limitation, amino acids, peptides, acylcarnitines, monosaccharides, lipids and phospholipids, prostaglandins, hydroxyeicosatetraenoic acids, hydroxyoctadecadienoic acids, steroids, bile acids, and glycolipids and phospholipids. Exemplary metabolites can be sphingolipids, glycosphingolipids, sphingosine, ceramide, sphingomyelin, sphingosylphosphorylcholin, dihydrosphingosine, phoshatidylcholine, phosphatidylinositol, phosphatidylserine, lysophoshatidylcholine, lysophosphatidylinositol, lysophosphatidylserine, plasmenylphoshatidylcholine, plasmanylphoshatidylcholine, proteinogenic amino acids, Alanine, Aspartic acid, Glutamic acid, Phenylalanine, Glycine, Histidine, Leucine, Isoleucine, Lysine, Methionine, Proline, Arginine, Serine, Threonine, Valine, Tryptophan, Tyrosine, asymmetrical dimethyl arginine, symmetrical dimethyl arginine, Glutamine, Asparagine, Nitrotyrosine, Hydroxyproline, Kynurenine, 3-Hydroxy kynurenine, non-proteinogenic amino acids, Ornithine, Citrulline, acylcarnitines, carnitine, free carnitine, acylcarnitine, hydroxylacylcarnitine, dicarboxylacylcarnitines, reducing monosaccharides, hexose, pentose, deoxyhexose, creatinine, creatine, spermidine spermine, putrescine, dopamine, serotonin, prostaglandins, hydoxyeicosatetraeneoic acid, Hydroxyoctadecadienoic acid, leukatrienes, thromboxanes, bile acids, sterols, cholesterols, vitamins and cofactors, drugs, and drug metabolites.

In certain embodiments of the invention, a sample may comprise one or more stabilizers for a cell or an analyte such as DNA, RNA, protein and/or lipid. For example, a sample may comprise a DNA stabilizer, an RNA stabilizer, and/or a protein stabilizer. Stabilizers are well known in the art and include, for example, DNAse inhibitors, RNAse inhibitors, and protease inhibitors or equivalents thereof.

In certain embodiments of the invention, profiles of at least one or more markers of a disease or condition are compared. This comparison can be quantitative or qualitative. Quantitative measurements can be taken using any of the assays described herein. For example, sequencing, direct sequencing, random shotgun sequencing, Sanger dideoxy termination sequencing, targeted sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.

Quantitative comparisons can include statistical analyses such as t-test, ANOVA, Krustal-Wallis, Wilcoxon, Mann-Whitney, and odds ratio. Quantitative differences can include differences in the levels of markers between profiles or differences in the numbers of markers present between profiles, and combinations thereof. Examples of levels of the markers can be, without limitation, gene expression levels, nucleic acid levels, protein levels, lipid levels, and the like. Qualitative differences can include, but are not limited to, activation and inactivation, protein degradation, nucleic acid degradation, and covalent modifications.

In certain embodiments of the invention, the profile is a nucleic acid profile, a protein profile, a lipid profile, a carbohydrate profile, a metabolite profile, or a combination thereof. The profile can be qualitatively or quantitatively determined.

A nucleic acid profile can be, without limitation, a genotypic profile, a single nucleotide polymorphism profile, a gene mutation profile, a gene copy number profile, a DNA methylation profile, a DNA acetylation profile, a chromosome dosage profile, a gene expression profile, or a combination thereof.

The nucleic acid profile can be determined by any methods known in the art to detect genotypes, single nucleotide polymorphisms, gene mutations, gene copy numbers, DNA methylation states, DNA acetylation states, chromosome dosages. Exemplary methods include, but are not limited to, polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), co-amplification at lower denaturation temperature-PCR (COLD-PCR), multiplex PCR, allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, bisulfite-driven conversion of non-methylated cytosine to uracil, co-amplification at lower denaturation temperature-PCR (COLD-PCR), multiplex PCR, methyl-binding PCR analysis, or a combination thereof.

As used herein, the term "sequencing" is used in a broad sense and refers to any technique known in the art that allows the order of at least some consecutive nucleotides in at least part of a nucleic acid to be identified, including without limitation at least part of an extension product or a vector insert. Exemplary sequencing techniques include targeted sequencing, single molecule real-time sequencing, electron microscopy-based sequencing, transistor-mediated sequencing, direct sequencing, random shotgun sequencing, Sanger dideoxy termination sequencing, targeted sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof. In some embodiments, sequencing comprises an detecting the sequencing product using an instrument, for example but not limited to an ABI PRISM® 377 DNA Sequencer, an ABI PRISM® 310, 3100, 3100-Avant, 3730, or 373OxI Genetic Analyzer, an ABI PRISM® 3700 DNA Analyzer, or an Applied Biosystems SOLiD™ System (all from Applied Biosystems), a Genome Sequencer 20 System (Roche Applied Science), or a mass spectrometer. In certain embodiments, sequencing comprises emulsion PCR. In certain embodiments, sequencing comprises a high throughput sequencing technique, for example but not limited to, massively parallel signature sequencing (MPSS).

In further embodiments of the invention, a protein profile can be a protein expression profile, a protein activation profile, or a combination thereof. In some embodiments, a protein activation profile can comprise determining a phosphorylation state, an ubiquitination state, a myristoylation state, or a conformational state of the protein.

A protein profile can be detected by any methods known in the art for detecting protein expression levels, protein phosphorylation state, protein ubiquitination state, protein myristoylation state, or protein conformational state. In some embodiments, a protein profile can be determined by an immunohistochemistry assay, an enzyme-linked immunosorbent assay (ELISA), in situ hybridization, chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microscopy, microfluidic chip-based assays, surface plasmon resonance, sequencing, Western blotting assay, or a combination thereof.

In some embodiments of the disclosure, a lipid profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof. Further methods for analyzing lipid content in a biological sample are known in the art (See, e.g., Kang et al. (1992) Biochim. Biophys. Acta. 1128:267; Weylandt et al. (1996) Lipids 31 :977; J. Schiller et al. (1999) Anal. Biochem. 267:46; Kang et al. (2001) Proc. Natl. Acad. Sci. USA 98:4050; Schiller et al. (2004) Prog. Lipid Res. 43:499). One exemplary method of lipid analysis is to extract lipids from a biological sample (e.g. using chloroform-methanol (2:1, vol/vol) containing 0.005% butylated hydroxytoluene (BHT, as an antioxidant)), prepare fatty acid methyl esters (e.g., using 14% BF3-methanol reagent), and quantify the fatty acid methyl esters (e.g., by HPLC, TLC, by gas chromatography-mass spectroscopy using commercially available gas chromatographs, mass spectrometers, and/or combination gas chromatograph/mass spectrometers). Fatty acid mass is determined by comparing areas of various analyzed fatty acids to that of a fixed concentration of internal standard.

In some embodiments of the disclosure, a carbohydrate profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

In some embodiments of the disclosure, a metabolite profile can be determind by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

As used herein, the "difference" between different profiles detected by the methods of this invention can refer to different gene copy numbers, different DNA, RNA, protein, lipid, or carbohydrate expression levels, different DNA methylation states, different DNA acetylation states, and different protein modification states. The difference can be a difference greater than 1 fold. In some embodiments, the difference is a 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference. In some embodiments, the difference is any fold difference between 1-10, 2-10, 5-10, 10-20, or 10-100 folds.

In some embodiments, the difference is differential gene expression (DGE), e.g. DGE of circulating diseased cells vs. control cells (or the control bodily fluid sample). DGE can be measured as X = log₂(Y_{P}) - log₂(Y_{NP}). The DGE may be any number, provided that it is significantly different between the circulating diseased cells and the control cells (or the control bodily fluid sample). For example, a 2-fold increased in gene expression could be represented as X = log₂(Y_{P}) - log₂(Y_{NP}) = log₂(Y_{P}/Y_{NP}) = log₂(2) = 1, while a 2-fold decrease in gene expression could be represented as X = log₂(Y_{P}) - log₂(Y_{NP}) = log₂(Y_{P}/Y_{NP}) =log₂(1/2) = -1. Down-regulated genes have X < 0, while up-regulated genes have X > 0. See, e.g., Efron, J Am Stat Assoc 104:1015-1028 (2009).

A general principle of assays to detect markers involves preparing a sample or reaction mixture that may contain the marker (e.g., one or more of DNA, RNA, protein, polypeptide, carbohydrate, lipid, metabolite, and the like) and a probe under appropriate conditions and for a time sufficient to allow the marker and probe to interact and bind, thus forming a complex that can be removed and/or detected in the reaction mixture. These assays can be conducted in a variety of ways.

For example, one method to conduct such an assay would involve anchoring the marker or probe onto a solid phase support, also referred to as a substrate, and detecting target marker/probe complexes anchored on the solid phase at the end of the reaction. In one embodiment of such a method, a sample from a subject, which is to be assayed for presence and/or concentration of marker, can be anchored onto a carrier or solid phase support. In another embodiment, the reverse situation is possible, in which the probe can be anchored to a solid phase and a sample from a subject can be allowed to react as an unanchored component of the assay.

There are many established methods for anchoring assay components to a solid phase. These include, without limitation, marker or probe molecules which are immobilized through conjugation of biotin and streptavidin. Such biotinylated assay components can be prepared from biotin-NHS(N-hydroxy-succinimide) using techniques known in the art (e.g., biotinylation kit, Pierce Chemicals, Rockford, IL), and immobilized in the wells of streptavidin-coated 96 well plates (Pierce Chemical). In certain embodiments, the surfaces with immobilized assay components can be prepared in advance and stored.

Other suitable carriers or solid phase supports for such assays include any material capable of binding the class of molecule to which the marker or probe belongs. Well known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non-immobilized component is added to the solid phase upon which the second component is anchored. After the reaction is complete, uncomplexed components may be removed (e.g., by washing) under conditions such that any complexes formed will remain immobilized upon the solid phase. The detection of marker/probe complexes anchored to the solid phase can be accomplished in a number of methods outlined herein.

In certain exemplary embodiments, the probe, when it is the unanchored assay component, can be labeled for the purpose of detection and readout of the assay, either directly or indirectly, with detectable labels discussed herein and which are well-known to one skilled in the art.

It is also possible to directly detect marker/probe complex formation without further manipulation or labeling of either component (marker or probe), for example by utilizing the technique of fluorescence energy transfer (see, for example, U.S. Patent Nos. 5,631,169 and 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determination of the ability of a probe to recognize a marker can be accomplished without labeling either assay component (probe or marker) by utilizing a technology such as real-time Biomolecular Interaction Analysis (BIA) (see, e.g., Sjolander, S. and Urbaniczky, C, 1991, Anal. Chem. 63:2338 2345 and Szabo et al, 1995, Curr. Opin. Struct. Biol. 5:699 705). As used herein, "BIA" or "surface plasmon resonance" is a technology for studying biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another embodiment, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas and Minton (1993) Trends Biochem. Sci. 18:284). Standard chromatographic techniques may also be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, e.g., Heegaard (1998) J. Mol. Recognit. 11 :141; Hage and Tweed (1997) J. Chromatogr. B. Biomed. Sci. Appl. 12:499). Gel electrophoresis may also be employed to separate complexed assay components from unbound components (see, e.g., Ausubel et al, ed., Current Protocols in Molecular Biology, John Wiley & Sons, New York, 1987 1999). In this technique, protein or nucleic acid complexes are separated based on size or charge, for example. In order to maintain the binding interaction during the electrophoretic process, non-denaturing gel matrix materials and conditions in the absence of reducing agent are typically preferred. Appropriate conditions to the particular assay and components thereof will be well known to one skilled in the art.

In certain exemplary embodiments, the level of mRNA corresponding to the marker can be determined either by *in situ* and/or by *in vitro* formats in a biological sample using methods known in the art. Many expression detection methods use isolated RNA. For in vitro methods, any RNA isolation technique that does not select against the isolation of mRNA can be utilized for the purification of RNA from blood cells (see, e.g., Ausubel et al, ed., Current Protocols in Molecular Biology, John Wiley & Sons, New York 1987 1999). Additionally, large numbers of cells and/or samples can readily be processed using techniques well known to those of skill in the art, such as, for example, the single-step RNA isolation process of Chomczynski (1989, U.S. Patent No. 4,843,155).

Isolated mRNA can be used in hybridization or amplification assays that include, but are not limited to, Southern or Northern analyses, polymerase chain reaction analyses and probe arrays. In certain exemplary embodiments, a diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to an mRNA or genomic DNA encoding a marker used in the present invention. Other suitable probes for use in the diagnostic assays of the invention are described herein. Hybridization of an mRNA with the probe indicates that the marker in question is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in a gene chip array. A skilled artisan can readily adapt known mRNA detection methods for use in detecting the level of mRNA encoded by the markers used in the present invention.

An alternative method for determining the level of mRNA corresponding to a marker of the present invention in a sample involves the process of nucleic acid amplification, e.g., by RT-PCR (the experimental embodiment set forth in U.S. Patent Nos. 4,683,195 and 4,683,202), co-amplification at lower denaturation temperature-PCR (COLD-PCR) (Li et al. (2008) Nat. Med. 14:579), ligase chain reaction (Barany, 1991, Proc. Natl. Acad. Sci. USA, 88:189), self sustained sequence replication (Guatelli et al., 1990, Proc. Natl. Acad. Sci. USA 87:1874), transcriptional amplification system (Kwoh et al. (1989) Proc. Natl. Acad. Sci. USA 86:1173), Q- Beta Replicase (Lizardi et al. (1988) Bio/Technology 6:1197), rolling circle replication (U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated from the sample (*e.g.,* a bodily fluid (*e.g.,* blood cells)) prior to detection. In such methods, a cell or tissue sample is prepared/processed using known histological methods. The sample is then immobilized on a support, typically a glass slide, and then contacted with a probe that can hybridize to mRNA that encodes the marker.

As an alternative to making determinations based on the absolute expression level of the marker, determinations may be based on the normalized expression level of the marker. Expression levels are normalized by correcting the absolute expression level of a marker by comparing its expression to the expression of a gene that is not a marker, *e.g.,* a housekeeping gene that is constitutively expressed. Suitable genes for normalization include housekeeping genes such as the actin gene, or epithelial cell- specific genes. This normalization allows the comparison of the expression level in a patient sample from one source to a patient sample from another source, e.g., to compare a circulating diseased cell from an individual to a control cell from the same individual.

In one embodiment of this invention, a protein or polypeptide corresponding to a marker is detected. In certain embodiments, an agent for detecting a protein or polypeptide can be an antibody capable of binding to the polypeptide, such as an antibody with a detectable label. As used herein, the term "labeled," with regard to a probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. Antibodies can be polyclonal or monoclonal. An intact antibody, or a fragment thereof (*e.g.,* Fab or F(ab')2) can be used. In one format, antibodies, or antibody fragments, can be used in methods such as Western blots or immunofluorescence techniques to detect the expressed proteins. In such uses, it is generally preferable to immobilize either the antibody or proteins on a solid support. Suitable solid phase supports or carriers include any support capable of binding an antigen or an antibody. Well known supports or carriers include glass, polystyrene, polypropylene, polyethylene, dextran, nylon, amylases, natural and modified celluloses, polyacrylamides, gabbros, magnetite and the like.

A variety of formats can be employed to determine whether a sample contains a protein that binds to a given antibody. Examples of such formats include, but are not limited to, competitive and non-competitive immunoassay, enzyme immunoassay (EIA), radioimmunoassay (RIA), antigen capture assays, two-antibody sandwich assays, Western blot analysis, enzyme linked immunoabsorbant assay (ELISA), a planar array, a colorimetric assay, a chemiluminescent assay, a fluorescent assay, and the like. Immunoassays, including radioimmmunoassays and enzyme- linked immunoassays, are useful in the methods of the present invention. A skilled artisan can readily adapt known protein/antibody detection methods for use in determining whether cells (e.g., bodily fluid cells such as blood cells) express a marker used in the present invention.

One skilled in the art will know many other suitable carriers for binding antibody or antigen, and will be able to adapt such support for use with the present invention. For example, protein isolated from cells (e.g., bodily fluid cells such as blood cells) can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

In certain exemplary embodiments, assays are provided for diagnosis, prognosis, assessing the risk of developing a disease, assessing the efficacy of a treatment, monitoring the progression or regression of a disease, and identifying a compound capable of ameliorating or treating a disease. An exemplary method for these methods involves obtaining a bodily fluid sample from a test subject and contacting the bodily fluid sample with a compound or an agent capable of detecting one or more of the markers of the disease or condition, *e.g.,* marker nucleic acid (*e.g.,* mRNA, genomic DNA), marker peptide (*e.g.,* polypeptide or protein), marker lipid (*e.g.,* cholesterol), or marker metabolite (*e.g.,* creatinine) such that the presence of the marker is detected in the biological sample. In one embodiment, an agent for detecting marker mRNA or genomic DNA is a labeled nucleic acid probe capable of hybridizing to marker mRNA or genomic DNA. The nucleic acid probe can be, for example, a full-length marker nucleic acid or a portion thereof. Other suitable probes for use in the diagnostic assays of the invention are described herein.

As used herein, a compound capable of ameliorating or treating a disease or condition can include, without limitations, any substance that can improve symptoms or prognosis, prevent progression of the disease or condition, promote regression of the disease or condition, or eliminate the disease or condition.

The methods of the disclosure can also be used to detect genetic alterations in a marker gene, thereby determining if a subject with the altered gene is at risk for developing a disease and/or disorder associated with cancer and/or an infectious agent, and/or one or more other disorders described herein characterized by misregulation in a marker protein activity or nucleic acid expression, such as cancer. In certain embodiments, the methods include detecting, in circulating diseased cells from the subject, the presence or absence of a genetic alteration characterized by an alteration affecting the integrity of a gene encoding a marker peptide and/or a marker gene. For example, such genetic alterations can be detected by ascertaining the existence of at least one of: 1) a deletion of one or more nucleotides from one or more marker genes; 2) an addition of one or more nucleotides to one or more marker genes; 3) a substitution of one or more nucleotides of one or more marker genes, 4) a chromosomal rearrangement of one or more marker genes; 5) an alteration in the level of a messenger RNA transcript of one or more marker genes; 6) aberrant modification of one or more marker genes, such as of the methylation pattern of the genomic DNA; 7) the presence of a non-wild type splicing pattern of a messenger RNA transcript of one or more marker genes; 8) a non-wild type level of a one or more marker proteins; 9) allelic loss of one or more marker genes; and 10) inappropriate post-translational modification of one or more marker proteins. As described herein, there are a large number of assays known in the art which can be used for detecting alterations in one or more marker genes.

In certain embodiments, detection of the alteration involves the use of a probe/primer in a polymerase chain reaction (PCR) (see, e.g., U.S. Patent Nos. 4,683,195, 4,683,202 and 5,854,033), such as real-time PCR, COLD-PCR (Li et al. (2008) Nat. Med. 14:579), anchor PCR, recursive PCR or RACE PCR, or, alternatively, in a ligation chain reaction (LCR) (see, e.g., Landegran et al. (1988) Science 241:1077; Prodromou and Pearl (1992) Protein Eng. 5:827; and Nakazawa et al. (1994) Proc. Natl. Acad. Sci. USA 91:360), the latter of which can be particularly useful for detecting point mutations in a marker gene (see Abravaya et al. (1995) Nucleic Acids Res. 23:675). This method can include the steps of collecting a sample of circulating diseased cells from a subject, isolating nucleic acid (e.g., genomic, mRNA or both) from the sample, contacting the nucleic acid sample with one or more primers which specifically hybridize to a marker gene under conditions such that hybridization and amplification of the marker gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli et al., (1990) Proc. Natl. Acad. Sci. USA 87:1874), transcriptional amplification system (Kwoh et al., (1989) Proc. Natl. Acad. Sci. USA 86:1173), Q Beta Replicase (Lizardi et al. (1988) Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in one or more marker genes from a sample can be identified by alterations in restriction enzyme cleavage patterns. For example, sample and control DNA is isolated, optionally amplified, digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Pat. No. 5,498,531) can be used to score for the presence of specific mutations by development or loss of a ribozyme cleavage site.

In other embodiments, genetic mutations in one or more of the markers described herein can be identified by hybridizing a sample and control nucleic acids, e.g., DNA or RNA, to high density arrays containing hundreds or thousands of oligonucleotides probes (Cronin et al. (1996) Human Mutation 7: 244; Kozal et al. (1996) Nature Medicine 2:753). For example, genetic mutations in a marker nucleic acid can be identified in two dimensional arrays containing light-generated DNA probes as described in Cronin, M. T. et al. supra. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence a marker gene and detect mutations by comparing the sequence of the sample marker gene with the corresponding wild-type (control) sequence. Examples of sequencing reactions include those based on techniques developed by Maxam and Gilbert ((1977) Proc. Natl. Acad. Sci. USA 74:560) or Sanger ((1977) Proc. Natl. Acad. Sci. USA 74:5463). It is also contemplated that any of a variety of automated sequencing procedures can be utilized when performing the diagnostic assays ((1995) Biotechniques 19:448), including sequencing by mass spectrometry (see, e.g., PCT International Publication No. WO 94/16101; Cohen et al. (1996) Adv. Chromatogr. 36:127-162; and Griffin et al. (1993) Appl. Biochem. Biotechnol. 38:147).

Other methods for detecting mutations in a marker gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes formed by hybridizing (labeled) RNA or DNA containing the wild-type marker sequence with potentially mutant RNA or DNA obtained from a tissue sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl. Acad. Sci. USA 85:4397; Saleeba et al. (1992) Methods Enzymol. 217:286. In one embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in marker cDNAs obtained from samples of cells. For example, the mutY enzyme of E. coli cleaves A at G/A mismatches and the thymidine DNA glycosylase from HeLa cells cleaves T at G/T mismatches (Hsu et al. (1994) Carcinogenesis 15:1657). According to an exemplary embodiment, a probe based on a marker sequence, e.g., a wild-type marker sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations in marker genes. For example, single strand conformation polymorphism (SSCP) may be used to detect differences in electrophoretic mobility between mutant and wild type nucleic acids (Orita et al. (1989) Proc. Natl. Acad. Sci. USA 86:2766, see also Cotton (1993) Mutat. Res. 285:125; and Hayashi (1992) Genet. Anal. Tech. Appl. 9:73). Single-stranded DNA fragments of sample and control marker nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility (Keen et al. (1991) Trends Genet. 7:5).

In yet another embodiment the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys. Chem. 265:12753).

Examples of other techniques for detecting point mutations include, but are not limited to, selective oligonucleotide hybridization, selective amplification or selective primer extension. For example, oligonucleotide primers may be prepared in which the known mutation is placed centrally and then hybridized to target DNA under conditions which permit hybridization only if a perfect match is found (Saiki et al. (1986) Nature 324:163; Saiki et al. (1989) Proc. Natl. Acad. Sci. USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucl. Acids Res. 17:2437) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci. USA 88:189). In such cases, ligation will occur only if there is a perfect match at the 3' end of the 5' sequence making it possible to detect the presence of a known mutation at a specific site by looking for the presence or absence of amplification.

In yet another aspect, this disclosure provides a method for identifying a compound capable of ameliorating or treating a disease or condition in a subject comprising: a) determining a first profile of one or more markers of the disease or condition from a population of circulating diseased cells from the subject before administering the compound to the subject; determining a second profile of at least one of the one or more markers from a population of control cells or a control bodily fluid sample from the subject before administering the compound to the subject; identifying a first difference between the first and second profiles of at least one or more of said markers; b) determining a third profile of the one or more markers from a population of circulating diseased cells from the subject after the administration of the compound; determining a fourth profile of at least one of the one or more markers from a population of control cells or a control bodily fluid sample from the subject after the administration of the compound; identifying a second difference between the third and fourth profiles of at least one or more of said markers; c) identifying a difference between the first difference and the second difference, wherein the identified difference indicates that the compound is capable of ameliorating or treating said disease or condition in the subject.

In yet another aspect, this disclosure provides a method for identifying one or more markers of a disease or condition comprising: determining a first profile of analytes from circulating diseased cells from a subject having said disease or condition; determining a second profile of analytes from control cells or a control bodily fluid sample from the subject having said disease or condition; and identifying one or more analytes specific to the first profile relative to the second profile, the identified analytes being markers of said disease or condition.

In yet another aspect, the one or more markers identified by this disclosure may be used in the treatment of a disease or condition. For example, a marker (e.g., protein or gene) identified by methods of the disclosure may be used as a molecular target for a therapeutic agent. A marker identified by the disclosure also may be used in any of the other methods of the invention, e.g., for monitoring the progression or regression of a disease or condition. In certain embodiments, the one or more markers identified by the methods of this disclosure may have therapeutic potential. For example, if a marker is identified as being up-regulated (or down-regulated) or activated (or inhibited) in circulating diseased cells from a subject having a disease or condition, a compound or an agent that is capable of down-regulating (or up-regulating) or inhibiting (or activating) said marker may be useful in treating said disease or condition. Similarly, a gene/protein/lipid/carbohydrate expression profile, a single nucleotide polymorphism profile, a gene mutation profile, a gene copy number profile, a DNA methylation profile, a DNA acetylation profile, a chromosome dosage profile, a gene expression profile, or a combination thereof may be useful in this aspect of the disclosure.

An exemplary method for detecting the presence or absence of an analyte (*e.g.*, DNA, RNA, protein, polypeptide, carbohydrate, lipid or the like) corresponding to a marker used in the invention in a biological sample involves obtaining a bodily fluid sample (*e*.*g*., blood) or cells isolated from a bodily fluid sample from a test subject and contacting the bodily fluid sample or the isolated cells with a compound or an agent capable of detecting one or more markers. Detection methods described herein can be used to detect one or more markers in a biological sample *in vitro* as well as *in vivo.* For example, *in vitro* techniques for detection of mRNA include Northern hybridizations and in situ hybridizations. *In vitro* techniques for detection of a polypeptide corresponding to a marker of the invention include enzyme linked immunosorbent assays (ELISAs), Western blots, immunoprecipitations and immunofluorescence. *In vitro* techniques for detection of genomic DNA include Southern hybridizations. Furthermore, *in vivo* techniques for detection of a polypeptide corresponding to a marker used in the invention include introducing into a subject a labeled antibody directed against the polypeptide. For example, the antibody can be labeled with a radioactive marker whose presence and location in a subject can be detected by standard imaging techniques. Because each marker is also an analyte, any method described herein to detect the presence or absence of a marker can also be used to detect the presence or absence of an analyte.

The marker that is useful in the methods of the invention can include any mutation in any one of the above-identified markers. Mutation sites and sequences can be identified, for example, by databases or repositories of such information, *e.g.*, The Human Gene Mutation Database (www.hgmd.cf.ac.uk), the Single Nucleotide Polymorphism Database (dbSNP, www.ncbi.nlm.nih.gov/projects/SNP), and the Online Mendelian Inheritance in Man (OMIM) website (www.ncbi.nlm.nih.gov/omim).

The marker that is useful in the methods of the invention can include any marker that is known to be associated with a disease or condition. Markers that can be used in this invention can be any marker that has been well-characterized as associated with a specific disease or condition, or any markers that have bee identified by the methods of this disclosure.

In some embodiments, the markers comprise at least one gene selected from the group consisting of AKT2, BAK1, EGFR, ERBB2, ETS2, FOS, JUN, MAP2K1, MMP2, PDGFB, RBI, SERPINB2, SNCG, and SPP1. In some embodiments, the one or more markers comprise at least one gene selected from the group consisting of AKT1, AKT2, BAK2, CDC25A, E2F1, EGFR, ERBB2, FOS, JUN, MAP2K1, MMP2, NFKB1, PDGFB, PIK3R1, PNN, RBI, SERPINB2, SERPINB5, SNCG, SPP1, TERT, TIMP3, and TP53. In some embodiments, the one or more markers comprise at least one gene selected from the group consisting of CASP8, CASP9, COL18A1, ETS2, HTATIP2, MMP9, SRC, and TWIST1. In some embodiments, the one or more markers comprise at least one gene selected from the group consisting of AKT1, APAF1, ATM, CDC25A, CDKN1A, ETS2, FOS, IL8, ITGA4, ITGA6, ITGAV, JUN, MAP2K1, NFKBIA, PLAU, PLAUR, RAF1, SERPINB2, SYK, TIMP1, TNF, TNFRSF10B, and TNFRSF1A. In some embodiments, the markers comprise at least one gene selected from the group consisting of ACP2, AK2, AKT3, ARL5B, ATP2B3, BGN, BRAF, BTG2, CAMKK2, CAPG, CAPN12, CPLX2, DENND5A, DNA2, FAM104A, FNIP1, GFRA4, GLUD1, GNAQ, GP1BB, HNRPLL, HOXA2, HPS3, INPP4A, ITGAV, KLHL23, LANCL2, LYPD6, MAPKAPK3, MEF2A (includes, EG:4205), MEF2C, NVL, PCYT1A, PGLYRP4, PLOD1, PPP1CB, PRKAB2, PROS1, PTPRE, RASA4 (includes,EG:10156), RBMS2, RBPJ, STAT5B, THBS1, TRIB1, TRIM2, TSPAN6, and ZDHHC21. In some embodiments, the markers comprise at least one gene selected from the group consisting of B4GALT5, BOP1, CCL2, CCL3, CCL3L1, CCRL2, CD83, CLEC4G, CLIC4, CTSC, CTSO, CXCL10, FCGR3A, FPR3, HBA1, HBB, LRMP, MAP1LC3B2, MS4A4A, MSR1, MYADML, NIDI, PF4, PION, RNF217, SAMD9L, SERPING1, and SPARC. In some embodiments, the markers comprise at least one gene selected from the group consisting of ACOT9, AMPD2, ARHGAP15, BATF2, C3AR1, C5orf41, CCL3, CCL3L1, CD63, CHST11, CHSY1, CLEC4G, CTSZ, CXorf21, CYTH4, CYTIP, DLEU2, DNAJA1, DOCK8, DTX3L, DUSP6, EPSTI1, ERF, F2RL1, FYB, GABRB2, GBP5, GLRX, GNB4, ICAM1, IFI35, IFIH1, IFNAR2, IL1R1, IRF1, ITGA5, LAP3, LAPTM5, LCP2, MAPILC3B, MAP1LC3B2, MICAL2, MT1DP, MT1JP, MT1M, MT2A, MYADML, NEK6, NINJ2, NNMT, NT5C3L, NUB1, PDE4B, PLOD1, PML, PRKCB, PSMB9, RCN3, RGS4, RNASE6, RTP4, SAMD9L, SEL1L, SERPING1, SETX, SIGLEC10, SKIL, SLC7A7, SNORA21, SP100, SP110, SP140, SSFA2, STAT2, STK17B, STK3, TDRD7, TMCC1, TMPRSS11E2, TNFRSF1B, TPM1, TRIM21,TXNDC4, UBE2L6, UBE2W, USP18, VAV1, WARS, WIPF1, and WIPI1. In some embodiments, the markers comprise at least one gene selected from the group consisting of ADAR, ADM, ALAS1, ANKRD22, ARHGAP27, B3GNT5, BCL10, C12orf35, C15orf29, C2orf59, CD177, CEACAM1, CPEB2, DDX58, F2RL1, GDPD3, GNAI3, HIST2H3A, HIST2H3D, HIST2H4A, HMGCR, HSPA6, HSPC159, IL4R, IMPA2, KPNB1, KREMEN1, KRT23, LDLR, LOC100130904, LTB4R, MAEA, MARK2, MBOAT2, MPZL3, N4BP1, NBEAL2, NMI, NPEPPS, PARP14, PGM2, PPIF, PXN, RALBP1, ROD1, RPS6KA1, S100P, SERTAD2, SLC9A1, SLPI, SP110, SPINT1, ST14, TBC1D3, TNFRSF9, TRIM21, UPP1, VPS24, ZBTB34, and ZNF256.

In some embodiments, the markers comprise at least one biomarker selected from the group consisting of ACTN4, BC020163, CMIP, CNN2, EDNRB, GPM6B, KIT, MGC40222, NAMPT, PRAME, RPL18, RPL21, RPS15, TMEM80, TRIB2, TTC3, and VDAC1. In one embodiment, the markers are ACTN4, BC020163, CMIP, CNN2, EDNRB, GPM6B, KIT, MGC40222, NAMPT, PRAME, RPL18, RPL21, RPS15, TMEM80, TRIB2, TTC3, and VDAC1. These markers are useful in the diagnosis, prognosis, or monitoring of melanoma, or discriminating between different types of skin lesions, for example, melanoma and naevi (See, e.g., Wachsman et al., "Noninvasive genomic detection of melanoma," Br J Dermatol. 2011 Apr;164(4):797-806.).

In some embodiments, the marker that is useful in the methods of the invention for prenatal or pregnancy-related diseases or conditions include those disclosed in, for example, United States Patents 7,655,399, 7,651,838, 6,660,477, 6,172,198, 5,594,637, 5,514,598, 6,258,540, 6,664,056, 7,235,359, and 7,645,576, United States Patent Application Publications 20090162842, 20090155776, 20070207466, 20060019278, 20040086864, 20020045176, 20010051341, 20020192642, 20040009518, 20040203037, 20050282185, 20060252071, 20070275402, 20080153090, 20090170102, 20090061425, 20020045176, 20040137452, 20050164241, 20060019278, 20060252068, 20060252071, 20060257901, 20070141625, 20070218469, 20070275402, 20090155776, 20090162842, 20090170102, 20090317797, 20100120056, 20100120076, and 20100137263 and International Patent Application Publications WO/2006/026020, WO/2002/068685, WO/2005/111626, WO/2009/055487, WO/2009/001392, and WO/2008/014516.

In some embodiments, the marker that is useful in the methods of the invention for neurological or neuropsychiatric diseases or conditions include those disclosed in, for example in United States Patents 7,723,117, 6,867,236, United States Patent Application Publications 20060115854, 20060115855, 20060166283, 20060234301, 20060259990, 20060259991, 20070162983, 20070264197, 20080026405, 20080038730, 20080051334, 20080152589, 20080220013, 20080261226, 20080269103, 20080286263, 20090041862, 20090239241, 20090275046, 20090318354, 20090324611, 20100009352, 20100021929, 20100028356, 20100055722, 20100062463, 20100075891, 20100105623, 20100124756, 20100159486, 20100167937, 20100169988, 20100167320, 20100112587, 20100098705, 20100068705, 20100009356, 20090305265, 20100124746, 20100092983, 20070148661, 20070141625, 20100120050, 20090155230, 20090274709, International Patent Application Publications WO/2004/040016, WO/2004/071269, WO/2005/033341, WO/2005/052592, WO/2005/103712, WO/2005/114222, WO/2006/020269, WO/2006/048778, WO/2006/050475, WO/2006/061609, WO/2006/105907, WO/2006/133423, WO/2006/134390, WO/2007/098585, WO/2007/119179, WO/2008/010660, WO/2008/014314, WO/2008/028257, WO/2008/046509, WO/2008/046510, WO/2008/046511, WO/2008/046512, WO/2008/063369, WO/2008/085035, WO/2008/095261, WO/2008/100596, WO/2008/120684, WO/2008/125651, WO/2008/127317, WO/2008/132464, WO/2009/000520, WO/2009/001392, WO/2009/068591, WO/2009/074331, WO/2009/100131, WO/2010/005750, WO/2010/011506, WO/2010/019553, WO/2010/059242, WO/2010/061283, WO/2010/063009, WO/2010/066000, WO/2009/121152, WO/2009/121951, WO/2009/097450, WO/2009/092382, WO/2009/075579, WO/2009/058168, WO/2009/053523, WO/2009/034470, WO/2009/032722, WO/2009/014639, WO/2009/003142, WO/2010/041046, WO/2007/131345, WO/2008/003826, and WO/2009/07556.

In some embodiments, the marker that is useful in the methods of the invention for cardiovascular diseases or conditions include those disclosed in, for example in United States Patents 7,670,769, 7,445,886, 7,432,107, 7,157,235, and 7,009,038, United States Patent Application Publications 20100167320, 20100112587, 20100098705, 20100068705, 20100009356, 20090305265, 20100124746, 20100092983, 20070148661, 20070141625, 20100120050, 20090155230, and 20090274709, and International Patent Application Publications WO/2009/121152, WO/2009/121951, WO/2009/097450, WO/2009/092382, WO/2009/075579, WO/2009/058168, WO/2009/053523, WO/2009/034470, WO/2009/032722, WO/2009/014639, WO/2009/003142, WO/2010/041046, WO/2007/131345, WO/2008/003826, and WO/2009/075566.

In some embodiments, the marker that is useful in the methods of the invention for kidney-associated diseases or conditions include those disclosed in, for example in United States Patents 7,488,584, 7,459,280, 7,294,465, and 7,662,578, United States Patent Application Publications 20100143951, 20100124746,20100120056,20100120041,20100081142,20090155230, and 20090239242, International Patent Application Publications WO/2010/059996, WO/2010/054389, WO/2010/048347, WO/2010/048497, WO/2010/054167, WO/2010/048346, WO/2010/046137, WO/2010/025434, WO/2010/018185, WO/2010/012306, WO/2009/122387, WO/2009/083950, WO/2009/080780, WO/2009/060035, WO/2009/059259, WO/2008/154238, WO/2008/089936, WO/2008/084331, WO/2008/042012, WO/2007/131345, WO/2005/012907, WO/2004/024098, WO/2003/019193, WO/2007/112999, WO/2007/082733, WO/2006/073941, WO/2010/068686, WO/2010/022210, and WO/2009/127644.

In some embodiments, the marker that is useful in the methods of the invention for autoimmune or immune-related diseases or conditions include those disclosed in, for example 7,604,948, 7,670,764, 6,986,995, and 6,631,330, United States Patent Application Publication 20070141625, 20090263474, 20100075891, 20100104579, 20100105086, 20100131286, 20090176217, 20090202469, 20020119118, 20090258025, 20100137393, 20100120629, 20090318392, 20090196927, 20090023166, 20080227709, 20080039402, 20080026378, 20070224638, 20070218519, 20060210562, 20050266432, 20050164233, 20050130245,20090130683,20090110667,20090054321,20090023166,and 20080274118, and International Patent Application Publication WO/2009/043848, WO/2010/053587, WO/2010/046503, WO/2010/039714, WO/2009/100342, WO/2009/053537, WO/2009/017444, WO/2008/156867, WO/2008/147938, WO/2008/129296, WO/2008/137835, WO/2008/082519, WO/2008/064336, WO/2008/043782, WO/2008/043725, WO/2007/047907, WO/2006/125117, WO/2006/114661, WO/2006/020899, WO/2005/114222, WO/2005/007836, WO/2004/076639, WO/2004/050704, and WO/2001/014881.

The present disclosure also provides kits that comprise marker detection agents that detect at least one or more of the markers identified by the methods of this invention. This present disclosure also provides methods of treating or preventing a disease or condition in a subject comprising administering to said subject an agent, a compound, or a protein that modulates the activity or expression, or disrupts the function of at least one or more of the markers identified by the methods of this disclosure.

The following examples are set forth as being representative of the present invention or disclosure. These examples are not to be construed as limiting the scope of the invention as these and other equivalent embodiments will be apparent in view of the present disclosure and accompanying claims.

### Example 1

### Representative Method I For The Separation Of Circulating Diseased Cells From A Whole Blood Sample

1. Separate plasma from whole blood (optional step).
2. Use magnetic antibody-conjugated beads (e.g., antibodies against epithelial cell adhesion molecule (EpCAM)-conjugated magnetic beads) to isolate circulating diseased cells (e.g., circulating tumor cells) from the remaining blood cells or from a whole blood sample.
3. Purify captured circulating diseased cells through a magnetic field.

### Example 2

### Representative Method II For The Separation Of Circulating Diseased Cells From A Whole Blood Sample

1. Separate blood sample into plasma and buffy coat including WBC sample. Stain WBC with fluorescent antibodies specific against the circulating diseased cell population (e.g., circulating tumor cells) and a DNA stain, (e.g., Hoechst 33342, Propidium iodide).
2. Sort the cells (e.g., by FACS).

### EXAMPLE 3 (comparative)

### Representative Method For The Analysis Of Expression Profiles

1. Isolate RNA from a population of circulating diseased cells and from a population of control cells (or a control bodily fluid). Prepare cDNA or cRNA and use to differentiate genetic profiles (e.g., a cancer gene array) between the circulating diseased cells and the control cells (or the control bodily fluid).
2. Isolate DNA from the circulating diseased cells and from the control cells (or the control bodily fluid). Run DNA arrays and compare the profiles obtained from the circulating diseased cells and the control cells (or the control bodily fluid).
3. Isolate protein from the circulating diseased cells and the control cells (or the control bodily fluid). Run Western blots using antibodies to known proteins overexpressed by human diseased cells/tissues (e.g., PSA and PSMA in prostate cancer; CEA in colon cancer; and CA125 in ovarian cancer), and compare the profiles obtained from the circulating diseased cells and the control cells (or the control bodily fluid).
4. Isolate lipids from the circulating diseased cells and the control cells (or the control bodily fluid). Compare quantity and quality of lipids, for example using HPLC, between the circulating diseased cells and the control cells (or the control bodily fluid).

## Claims

1. A method for diagnosing or aiding in the diagnosis of a disease or condition in a subject comprising:
a) determining a first profile of one or more markers of the disease or condition from a population of circulating diseased cells isolated from the subject, wherein the circulating diseased cells are affected by the disease or condition, and wherein the circulating diseased cells are blood cells;
b) determining a second profile of at least one of the one or more markers from a population of control cells or a control bodily fluid sample isolated from the subject, wherein the control cells or the control bodily fluid sample are at least 75% free of cells affected by the disease or condition; and
c) identifying a difference between the first and second profiles, wherein the difference is indicative of the presence of said disease or condition, or the risk of developing said disease or condition, or the prognosis of said disease or condition, in the subject.

2. The method of claim 1, wherein the one or more markers are nucleic acids, proteins, lipids, carbohydrates, metabolites, or combinations thereof.

3. The method of claims 1, wherein the profile is a nucleic acid profile, a protein profile, a lipid profile, a carbohydrate profile, a metabolite profile, or a combination thereof.

4. The method of claim 3, wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof.

5. The method of claim 3, wherein the protein profile is a protein expression profile, a protein activation profile, or a combination thereof.

6. The method of claim 5, wherein the protein activation profile comprises determining a phosphorylation state, an ubiquitination state, a myristoylation state, a conformational state, or a combination thereof of the one or more markers.

7. The method of any one of claims 1-6, wherein the disease or condition is a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a pediatric disease, disorder or condition, a mitochondrial disorder, a respiratory-gastrointestinal tract disease or condition, a reproductive disease or condition, an ophthalmic disease or condition, a musculo-skeletal disease or condition, or a dermal disease or condition.

## Patentansprüche

1. Verfahren zur Diagnose oder zur Unterstützung bei der Diagnose einer Erkrankung oder eines Zustandes in einem Individuum, das umfasst:
a) Bestimmen eines ersten Profils von einem oder mehreren Markern der Erkrankung oder des Zustandes von einer von dem Individuum isolierten Population zirkulierender erkrankter Zellen, wobei die zirkulierenden erkrankten Zellen von der Erkrankung oder dem Zustand betroffen sind, und wobei die zirkulierenden erkrankten Zellen Blutzellen sind;
b) Bestimmen eines zweiten Profils von mindestens einem des einen oder der mehreren Marker von einer Population an Kontrollzellen oder von einer Kontrollprobe einer Körperflüssigkeit, die von dem Individuum isoliert wurde(n), wobei die Kontrollzellen oder die Kontrollprobe der Körperflüssigkeit zu mindestens 75% frei von Zellen ist/sind, die von der Erkrankung oder dem Zustand betroffen sind;
c) Identifizierung des Unterschieds zwischen dem ersten und dem zweiten Profil, wobei der Unterschied auf das Vorliegen der Erkrankung oder des Zustandes, oder auf das Risiko, die Erkrankung oder den Zustand zu entwickeln, oder auf die Prognose der Erkrankung oder des Zustandes in dem Individuum hinweist.

2. Verfahren nach Anspruch 1, wobei der eine oder die mehreren Marker Nucleinsäuren, Proteine, Lipide, Kohlenhydrate, Metaboliten, oder Kombinationen davon sind.

3. Verfahren nach Anspruch 1, wobei das Profil ein Nucleinsäureprofil, ein Proteinprofil, ein Lipidprofil, ein Kohlenhydratprofil, ein Metabolitenprofil, oder eine Kombination davon ist.

4. Verfahren nach Anspruch 3, wobei das Nucleinsäureprofil ein genotypisches Profil, ein Profil eines einzelnen Nucleotidpolymorphismus, ein Genmutationsprofil, ein Profil der Genkopieanzahl, ein DNA-Methylierungsprofil, ein DNA-Acetylierungsprofil, ein Chromosomendosisprofil, ein Genexpressionsprofil, oder eine Kombination davon ist.

5. Verfahren nach Anspruch 3, wobei das Proteinprofil ein Proteinexpressionsprofil, ein Proteinaktivierungsprofil, oder eine Kombination davon ist.

6. Verfahren nach Anspruch 5, wobei das Proteinaktivierungsprofil umfasst: Bestimmen eines Phosphorylierungszustandes, eines Ubiquitinierungszustandes, eines Myristoylierungszustandes, eines Konformationszustandes, oder einer Kombination davon von dem einen oder den mehreren Markern.

7. Verfahren nach einem der Ansprüche 1 bis 6, wobei die Erkrankung oder der Zustand ist: ein(e) kardiovaskuläre(r) Erkrankung oder Zustand ein(e) mit Nieren in Zusammenhang stehende(r) Erkrankung oder Zustand, ein(e) pränatale(r) oder mit Schwangerschaft in Zusammenhang stehende(r) Erkrankung oder Zustand, ein(e) neurologische(r) oder neuropsychiatrische(r) Erkrankung oder Zustand, ein(e) autoimmune(r) oder immunbezogene(r) Erkrankung oder Zustand, ein Krebs, ein(e) infektiöse(r) Erkrankung oder Zustand, ein(e) pädiatrische(r) Erkrankung, Störung oder Zustand, eine mitochondriale Erkrankung, ein(e) Erkrankung oder Zustand des Atemweg-Gastrointestinaltrakts, ein(e) reproduktive(r) Erkrankung oder Zustand, ein(e) Erkrankung oder Zustand des Auges, ein(e) Erkrankung oder Zustand der Muskeln oder des Skeletts, oder ein(e) dermatologische(r) Erkrankung oder Zustand.

## Revendications

1. Procédé pour diagnostiquer ou aider à diagnostiquer une maladie ou une affection chez un sujet comprenant :
a) la détermination d'un premier profil d'un ou plusieurs marqueurs de la maladie ou de l'affection dans une population de cellules malades en circulation isolées à partir du sujet, dans lequel les cellules malades en circulation sont affectées par la maladie ou l'affection, et dans lequel les cellules malades en circulation sont des cellules sanguines ;
b) la détermination d'un second profil d'au moins l'un des un ou plusieurs marqueurs dans une population de cellules témoins ou un échantillon de fluide corporel témoin isolés à partir du sujet, dans lequel les cellules témoins ou l'échantillon de fluide corporel témoin sont au moins à 75 % exempts de cellules affectées par la maladie ou l'affection ; et
c) l'identification d'une différence entre les premier et second profils, dans lequel la différence indique la présence de ladite maladie ou affection, ou le risque de développer ladite maladie ou affection, ou le pronostic de ladite maladie ou affection, chez le sujet.

2. Procédé selon la revendication 1, dans lequel les un ou plusieurs marqueurs sont des acides nucléiques, des protéines, des lipides, des glucides, des métabolites, ou des combinaisons de ceux-ci.

3. Procédé selon les revendications 1, dans lequel le profil est un profil d'acide nucléique, un profil de protéine, un profil de lipide, un profil de glucide, un profil de métabolite, ou une combinaison de ceux-ci.

4. Procédé selon la revendication 3, dans lequel le profil d'acide nucléique est un profil génotypique, un profil de polymorphisme mononucléotidique, un profil de mutation génétique, un profil de nombre de copies de gène, un profil de méthylation de l'ADN, un profil d'acétylation de l'ADN, un profil de dosage chromosomique, un profil d'expression génétique, ou une combinaison de ceux-ci.

5. Procédé selon la revendication 3, dans lequel le profil de protéine est un profil d'expression protéique, un profil d'activation protéique, ou une combinaison de ceux-ci.

6. Procédé selon la revendication 5, dans lequel le profil d'activation protéique comprend la détermination d'un état de phosphorylation, d'un état d'ubiquitination, d'un état de myristoylation, d'un état conformationnel, ou d'une combinaison de ceux-ci, des un ou plusieurs marqueurs.

7. Procédé selon l'une quelconque des revendications 1 à 6, dans lequel la maladie ou l'affection est une maladie ou une affection cardiovasculaire, une maladie ou une affection associée au rein, une maladie ou une affection prénatale ou liée à la grossesse, une maladie ou une affection neurologique ou neuropsychiatrique, une maladie ou une affection auto-immune ou liée au système immunitaire, un cancer, une maladie ou une affection infectieuse, une maladie, un trouble ou une affection pédiatrique, un trouble mitochondrial, une maladie ou une affection du tractus respiratoire-gastro-intestinal, une maladie ou une affection de l'appareil reproducteur, une maladie ou une affection ophtalmique, une maladie ou une affection musculo-squelettique, ou une maladie ou une affection cutanée.
